# EUROPEAN PATENT SPECIFICATION

(11) **EP 4 076 411 B1**
(45) Date of publication and mention of the grant of the patent: **30.09.2026**
(21) Application number: 20829903.2
(22) Date of filing: 16.12.2020
(51) Int. Cl.: A61K 31/085, A61K 31/196, A61K 31/197, A61K 31/343, A61K 31/37, A61K 31/4152, A61K 31/4178, A61K 31/44, A61K 31/536, A61K 31/603, A61K 31/635, A61K 31/65, A61K 31/7048, A61K 31/7052, A61K 31/7056, A61K 45/06, A61P 1/00, A61P 1/14

(54) **DICUMAROL FOR USE FOR THE TREATMENT OF DYSBIOSIS AS AN ANTIDOT FOR MICROBIOME PROTECTION FROM MACROLIDE ANTIBIOTICS**
DICUMAROL ZUR VERWENDUNG ZUR BEHANDLUNG VON DYSBIOSE ALS ANTIDOT FÜR DEN MIKROBIOMSCHUTZ VOR MAKROLIDANTIBIOTIKA
DICUMAROL DESTINÉ À ÊTRE UTILISÉ POUR LE TRAITEMENT DE LA DYSBIOSE COMME ANTIDOT POUR LA PROTECTION DU MICROBIOME CONTRE LES ANTIBIOTIQUES MACROLIDES

(30) Priority: 16.12.2019 EP 19216548
(43) Date of publication of application: 26.10.2022
(73) Proprietor: European Molecular Biology Laboratory, 69117 Heidelberg (DE)
(72) Inventor: GONTAO BROCHADO, Ana, Rita, 97246 Eibelstadt (DE); TYPAS, Athanasios, 69120 Heidelberg (DE); MAIER, Lisa, 72076 Tübingen (DE); GOEMANS, Camilie, 69117 Heidelberg (DE); CACACE, Elisabetta, 53100 Siena (IT)
(74) Representative: Krauss, Jan
(86) International application number: PCT/EP2020/086511
(87) International publication number: WO 2021/122809

(56) References cited:
- EP-A1- 3 524 237
- WO-A1-2019/206781
- GARCÍA-LÓPEZ MARINA ET AL: "Analysis of 1,000 Type-Strain Genomes Improves Taxonomic Classification of Bacteroidetes", FRONTIERS IN MICROBIOLOGY, vol. 10, 23 September 2019 (2019-09-23), Lausanne, XP093282441, ISSN: 1664-302X, Retrieved from the Internet <URL:https://www.frontiersin.org/journals/microbiology/articles/10.3389/fmicb.2019.02083/full> DOI: 10.3389/fmicb.2019.02083
- CHAZ LANGELIER: "A proactive charcoal approach shields the gut microbiome from systemic antibiotics", SCIENCE TRANSLATIONAL MEDICINE, vol. 10, no. 430, 28 February 2018 (2018-02-28), pages eass8966, XP009520376
- DURANT: "Experiences with penicillin and dicumarol in the treatment of subacute bacterial endocarditis - Thill, C. J., and Meyer, O. O.: Am. J. M. Sc. 213:300 (March), 1947", AMERICAN HEART JOURNAL, ELSEVIER, AMSTERDAM, NL, vol. 34, no. 2, 1 August 1947 (1947-08-01), pages 300, XP022903982, ISSN: 0002-8703, [retrieved on 19470801], DOI: 10.1016/0002-8703(47)90329-3
- GIULIO QUARTA; SUBIN KIM; ILSEUNG CHO; MARTINBLASER: "Warfarin Induces Intestinal Dysbiosis Involving Vitamin K-Expressing Bacteria", GASTROENTEROLOGY, vol. 148, no. 4, Suppl.1, MO1815, 26 April 2015 (2015-04-26), pages S718, XP009520435

## Description

### FIELD OF THE INVENTION

The present invention relates to the field of therapeutics and, more in particular, to pharmaceutical compositions for the prevention and/or treatment of bacterial infections and antibacterial-induced dysfunctions. The invention is based on the identification of compounds that prevent antibacterial effects of antibiotics on bacterial species present in the human microbiome, while retaining antibacterial effects of antibiotics on pathogenic bacteria. The invention also pertains to pharmaceutical compositions comprising antibiotics and antibiotic antidotes for the protection of commensal gut bacteria. Thus, this invention relates to compounds and pharmaceutical combinations useful to prevent antibiotic-induced adverse effects on the gut microbiome.

### DESCRIPTION

Medication is emerging as major contributor for changes in the composition of the human gut microbiota, and antibiotics govern the risk of harming the healthy intestinal flora. Severe and long-lasting changes in the composition of the human gut microbiota are associated, and are in some cases even causatively linked, to dysbiosis and a wide range of diseases. Although several non-antibiotic drugs may also have a previously unappreciated impact on the gut microbiome composition, antibiotics, developed to have broad spectra and thereby target very diverse pathogens, are known to take a heavy toll on our gut flora, causing a variety of gastrointestinal side-effects, including *Clostridioides (former Clostridium) difficile* infections.

Recently, more attention has been given to this collateral damage of antibiotics on the gut microbiota and thereby, on the host's wellbeing with *in vivo* studies highlighting links between the long-term microbiota compositional changes and host dysbiosis, including the development of allergic, metabolic, immunological and inflammatory diseases. While uncovering the direct effects of different antibiotics on the gut flora is critical to improve general health, technical difficulties hamper routine testing of antibiotic susceptibility in anaerobes.

Currently available data on bacterial susceptibility to antibiotics offers little to no resolution in the diversity of the human gut microbiota, and information is missing even for the most prevalent and abundant species, or species associated with dysbiosis and disease. Current methods for retaining healthy microbiota upon antibiotic therapies suffer from many limitations and are difficult to investigate and improve.

WO2014197562 (A1) provides methods for microbiota restoration therapies based on collecting human fecal samples and performing fecal transplants.

WO0207741 (A1) provides methods for a probiotic recolonisation therapy comprising recolonizing a dysbiotic enteric microflora in a subject in need thereof by administering a pharmaceutical composition comprising viable non-pathogenic or attenuated pathogenic Clostridia, viable non-pathogenic or attenuated pathogenic Bacteroides, and viable non-pathogenic or attenuated pathogenic Escherichia coli. Said viable non-pathogenic or attenuated pathogenic bacteria are capable of recolonizing a dysbiotic enteric microflora in a subject.

Langelier *et al.,* 2018 (Langelier et al. A proactive charcoal approach shields the gut microbiome from systemic antibiotics. Science Translational Medicine; 28 Feb 2018) discloses a method for gut microbiota protection against antibiotics using co-administration of activated charcoal.

Pitout *et al.,* 2009 (Pitout et al. IPSAT P1A, a class A beta-lactamase therapy for the prevention of penicillin-induced disruption to the intestinal microflora. Curr. Opin. Investig. Drugs. 2009 Aug; 10(8):838-44) discloses a method for the protection of the gut microbiota against antibiotics using co-administration of beta-lactamase.

EP 3 524 237 A1 relates to repurposing pharmaceutical compounds for the treatment of infectious diseases, i.e. as antibiotics. Some act very specifically against particular bacterial species, therewith avoiding side effects, in particular on the microbiome; others have a strong broad-spectrum inhibitory effectiveness. Both types of compounds can further be used for modulating the microbiome composition, i.e. gut microbiota, and targeting species associated with dysbiosis. Dicumarol is particularly mentioned as effective in the modification of the growth of bacterial cells. This finds its application in the treatment against pathological species such as Clostridium, or in modulating the microbiome composition and targeting species associated with dysbiosis.

However, previous methods for retaining healthy microbiota upon antibiotic therapies are not very efficient. For example, the use of fecal transplants has many limitations, such as the invasive nature and no guarantee for colonization upon fecal transplantion. Moreover, many patients are reluctant to receiving fecal transplants, and methods based on the use of fecal samples suffer from reservation against collection. The use of proactive activated charcoal or β-lactamase for protection of the gut microbiota against antibiotics suffers from inconveniences, such as high costs due to handling and storage. Moreover, while activated charcoal and β-lactamases show activities to some specific antibiotics, activated charcoal and β-lactamases are not very active against multiple other antibiotics. Thus, the use of activated charcoal and β-lactamases is very limited.

Due to the continuing need for methods to protect the gut microbiota from the effect of antibiotics, it is an objection of the present invention to provide compounds and/or pharmaceutical compositions that selectively antagonize the effect of antibiotics on gut microbes. It is a further object of this invention to develop antibacterial therapies that can prevent an adverse impact of antibiotic compounds selectively on healthy microorganisms residing in the patients' body, such as the gut microbiota, while retaining antibiotic activity of antibiotic compounds against pathogens. It is an additional object of this invention to develop compounds and pharmaceutical compositions useful in selectively protecting the gut microbiota from the adverse effects of antibiotic therapies, and methods for using the same. Yet another object of this invention to develop therapies for treating dysbiosis.

### BRIEF DESCRIPTION OF THE INVENTION

The invention is set out in the appended set of claims. Any subject-matter falling outside the scope of the claims is provided for information, only.

### DETAILED DESCRIPTION OF THE INVENTION

In a first aspect thereof, the object of the present invention is solved by providing Dicumarol, or a pharmaceutically acceptable salt thereof, for use in treating dysbiosis and in antagonizing the antibacterial effect of at least one macrolide antibiotic, such as erythromycin, in *Phocaeicola,* such as, for example, *P. vulgatus,*
and wherein said dicumarol, or the pharmaceutically acceptable salt thereof, does not antagonize the antibacterial effect of said at least one macrolide antibiotic, such as erythromycin, in at least one second pathogenic bacterium, such as, for example, *S. aureus* or *S. pneumoniae.*

As used herein, "antagonism" or "antagonistic effect" refers to the effect that occurs when at least two compounds interact, and result in an overall effect that is lower than the sum of individual effects of either of them used alone. An "antagonistic" combination, as used herein, can prevent the antibacterial effect of one of the compounds used alone. Stated another way, antagonistic effect means that if at least one of the compounds is having an antibacterial effect against a bacterium or a bacterial species, this antibacterial effect can be counteracted by the at least one other compound. The at least one other compound can thus be seen as an "antidote" to said first compound. Particularly preferred is that the antagonistic effect occurs in a commensal bacterial species only, e.g. a human gut bacterial species, but does not occur in a pathogenic bacterial species. The combination of the at least two compounds is referred to as having an "antagonistic effect" if the antibacterial effect of at least one of the two compounds is masked when used in combination with the other compound in at least one bacterial species. "Antagonizing", in the context of the present invention, refers to having an "antagonistic effect". Stated another way, antagonistic effect means that if at least one of the compounds is having an antibacterial effect against a bacterium or a bacterial species, this antibacterial effect is counteracted, i.e. "antagonized", by the at least one other compound.

The term "synergy" or "synergistic effect" shall refer to the effect that occurs when at least two compounds interact, and result in an overall effect that is greater than the sum of individual effects of either of said compounds used alone. This combination thus greatly improves the antibacterial effect of one of the compounds used alone. Stated another way, synergistic effect means that the total antibacterial effect against a bacterium or a bacterial species of the combination of the two components is greater than the sum of the antibacterial effect of each component when measured separately.

The term "antibacterial" or "antibacterial effect" as used herein refers to the general term for bacteriostatic and bactericidal effects. The term "bacteriostatic" or "bacteriostatic action" refers to the inhibition of the growth and reproduction of microorganisms. As used herein, the term "bactericidal" or "bactericidal effect" shall refer to the role of killing microbial trophozoites and propagules. The term "antibacterial effective amount" as used herein refers to an antibacterial amount capable of achieving a desired antibacterial effect. Generally, depending on the antimicrobial requirements, the antimicrobial effects that need to be achieved may vary. The skilled artisan is aware of ways to determine the antibacterial effective amount of an antibacterial compound needed for achieving the desired antibacterial effect.

Importantly, many bacterial species residing in a person's or a patient's body are commensal or probiotic bacterial species. However, compounds having an antibiotic effect are often not only effective against pathogenic bacteria but also effective against commensal or probiotic bacterial species, thereby damaging the human or animal body, e.g. by harming the healthy gut microbiome. It is thus important to prevent such damaging effects on commensal and/or probiotic bacteria, while simultaneously enabling an antibiotic effect on pathogenic bacteria, which are causative for an infection and/or a disease.

The term "microbiota" refers, collectively, to the entirety of microbes found in association with a higher organism, such as a human. Organisms belonging to a human's microbiota may generally be categorized as bacteria, archaea, yeasts, and single-celled eukaryotes, as well as viruses and various parasites.

The term "microbiome" refers, collectively, to the entirety of microbes, their genetic elements (genomes), and environmental interactions, found in association with a higher organism, such as a human. Preferably, the term "microbiome" in the context of this invention shall refer to the commensal microbiome of a subject.

The microbiome comprises many commensal and/or probiotic bacterial strains. The term "commensal" refers to organisms that are normally harmless to a host, and can also establish mutualistic relations with the host. The human body contains about 100 trillion commensal organisms, which have been suggested to outnumber human cells by a factor of 10.

The term "probiotic" as used herein means living microorganisms, which when administered in adequate amounts, confer a health benefit on the host. Probiotics may be available in foods and dietary supplements (for example, but not limited to capsules, tablets, and powders). Examples of food containing probiotics are yogurt, fermented and unfermented milk, miso, tempeh, some juices and soy beverages. Some bacterial strains of the microbiome are known to have a probiotic function, such as *Lactobacillus, Bifidobacterium, Enterococcus, Streptococcus, Pediococcus, Leuconostoc, Bacillus, Escherichia, and Lactococcus.*

The term "dysbiosis" (also called dysbacteriosis) shall refer to any kind of imbalance of the microbiome. For example, species that are normally underrepresented in the microbiome of a healthy human being become overrepresented during the condition of dysbiosis, whereas normally dominated species of a healthy human being become underrepresented during the condition of dysbiosis. Most often, dysbiosis is a condition in the gastrointestinal tract, particularly during small intestinal bacterial overgrowth (SIBO) or small intestinal fungal overgrowth (SIFO). Dysbiosis has been reported to be associated with illnesses, such as inflammatory bowel disease, bacterial vaginosis, and colitis.

The inventors identified an antagonistic antibacterial effect of at least one compound on at least one first bacterium, wherein said first bacterium is a commensal bacterium and/or a probiotic bacterium, while said at least one compound is not having an antagonistic antibacterial effect on at least one second bacterium, wherein said second bacterium is a pathogenic bacterium. Thus, the at least one first compound is not preventing an antibacterial effect of the at least one second compound on a pathogenic bacterium, while the at least one first compound is preventing and/or relieving collateral damage to at least one commensal bacterium and/or a probiotic bacterium. The use of said at least one first compound in combination with said at least one second compound is thus advantageous over using one of the compounds alone, since the combination of compounds is preventing the damage of said compound having an antibacterial effect on a commensal bacterium and/or a probiotic bacterium.

In a preferred embodiment of this invention, said compound for use and said at least one second compound are administered to a subject simultaneously, separately, or sequentially. In particular, said at least one first compound and said at least one second compound are administered to a subject simultaneously, separately, or sequentially.

A further preferred embodiment relates to the compound for use of this invention, wherein said compound is administered to the subject by any suitable means including oral, intravenous, arterial infusion, intraperitoneal, parenteral, enteral, topical, intramuscular, subcutaneous, surgical, topical, cutaneous, subcutaneous, or any other clinically approved way of administration.

"Human-targeted drug", in the context of the present invention, shall refer to a compound intended for the use in humans. Preferably, the mechanism of action (MOA) of said drug is known and may affect a human cell intracellularly, extracellularly, or within the human cell membrane. The use of said human-targeted drug may suffer from the fact that it has side effects harming a human cell or organism. Examples of such human-targeted drugs include, without being limited thereto, antipsychotics, *anesthetics,* acid-reducing medications, chemotherapy drugs, and blood-pressure medications. Contrary, the term "antibiotic", in the context of the present invention, shall refer to a compound that is preferably microbiologically active, i.e. for use against pathogenic/undesired microbes.

Said at least one second compound is having an antibacterial effect on at least one bacterial species.

The term "antibiotic", as used herein, relates to a chemical substance, which at certain concentrations kills or prevents the growth of certain microorganisms, generally bacteria, although some antibiotics are also used for the treatment of infections caused by fungi or protozoa. Antibiotics are used in human, animal or horticultural medicine to treat infections caused by microorganisms.

Said at least one second compound is a macrolide antibiotic, preferably wherein said macrolide antibiotic is selected from erythromycin, azithromycin, clarithromycin, dirithromycin, and clindamycin, or a pharmaceutically acceptable salt thereof.

The compound for use is Dicumarol, or a pharmaceutically acceptable salt thereof, and said at least one second compound is a macrolide antibiotic, such as erythromycin, azithromycin, clarithromycin, dirithromycin, or clindamycin, preferably wherein said at least one second compound is erythromycin, or a pharmaceutically acceptable salt thereof.

In the context of this invention Dicumarol shall refer to 3,3'-Methylene-bis(4-hydroxycoumarin).

Preferred is that said at least one first bacterium is at least one bacterial species selected from *Phocaeicola vulgatus, Phocaeicola coprocola, and Phocaeicola dorei.*

Then, a further preferred embodiment relates to said at least one second bacterium, wherein said at least one second bacterium is a Gram-positive bacterium or a Gram-negative bacterium, and wherein said at least one second bacterium is a member of a genus selected from *Enterococcus, Staphylococcus, Enterobacter, Streptococcus, Pseudomonas, Escherichia, Salmonella, Helicobacter, Neisseria, Campylobacter, Chlamydia, Clostridia, Citrobacter, Vibrio, Treponema, Mycobacterium, Klebsiella, Actinomyces, Bacteroides, Bordetella, Borrelia, Brucella, Corynebacteria, Diplococcus, Fusobacterium, Leptospira, Listeria, Pasteurella, Proteus, Rickettsia, Shigella, Yersinia, Parabacteroides, Odoribacter, Faecalibacteria, Collinsella, Eggerthella, Lactonifactor, Pediococcus, Leuconostoc, Lactococcus, Roseburia, Coliform, Bacillus, Franscicella, Acinetobacter, Legionella, Actinobacillus, Coxiella, Kingella kingae, Haemophilus,* and combinations thereof, optionally wherein said second bacterium is an antibiotic-resistant bacterium and/or a multi-drug resistant bacterium.

In a further preferred embodiment of this invention, said at least one second bacterium is a pathogenic bacterium.

In the context of this invention, at least one bacterial species can be 1 bacterial species, or 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or any other number of bacterial species.

In a preferred embodiment, said compound for use is antagonizing said antibacterial effect of said at least one second compound on 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 bacterial species, or any number of bacterial species.

Preferred is a compound for use of this invention, wherein said at least one second compound is for use in the prevention and/or treatment of a bacterial infection, preferably wherein said bacterial infection is selected from an infection of the gastrointestinal tract, an infection of the urogenital tract, an infection of the upper and lower respiratory tract, rhinitis, tonsillitis, pharyngitis, bronchitis, pneumonia, an infection of the inner organs, nephritis, hepatitis, peritonitis, endocarditis, meningitis, osteomyelitis, an infection of the eyes, an infection of the ears, a cutaneous infection, a subcutaneous infection, an infection after burn, diarrhea, colitis, pseudomembranous colitis, a skin disorder, toxic shock syndrome, bacteremia, sepsis, pelvic inflammatory disease, an infection of the central nervous system, wound infection, intra-abdominal infection, intravascular infection, bone infection, joint infection, acute bacterial otitis media, pyelonephritis, deep-seated abscess, and tuberculosis.

The term "infection", as used in the context of the present invention, relates to the presence of bacteria, viruses, fungi, protozoa or other microorganisms, in or on a subject as well as the invasion by bacteria, viruses, fungi, protozoa or other microorganisms. The invasion includes undesired proliferation of pathogenic microbes in a host organism. More generally, a microbial infection can be any situation, in which the presence of a microbial population(s) is damaging to a host, such as a host animal. Thus, a microbial infection exists when excessive microorganisms are present in or on a mammal's body, or when the effects of the presence of a microbial population(s) is damaging the cells or other tissue of a mammal. Thus, the inhibition of the growth of such invading microorganisms results in a benefit to the subject that is infected by the microbial population(s). Examples of bacterial infections are urinary tract infection (UTI), kidney infections (pyelonephritis), gynecological and obstetrical infections, respiratory tract infection (RTI), acute exacerbation of chronic bronchitis (AECB), community-acquired pneumonia (CAP), hospital-acquired pneumonia (HAP), ventilator associated pneumonia (VAP), intra-abdominal pneumonia (IAI), acute otitis media, acute sinusitis, sepsis, catheterrelated sepsis, chancroid, chlamydia, skin infections, and bacteremia.

The term "treating" as used in the context of this invention means stabilizing or reducing an adverse symptom associated with a condition; reducing the severity of a disease symptom; slowing the rate of the progression of a disease; inhibiting or stabilizing the progression of a disease condition; or changing a metric that is associated with the disease state in a desirable way.

A further preferred embodiment relates to a compound for use of this invention, wherein said compound for use is in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, room spray, or combinations thereof.

The compounds for use of this invention are preferably administered to a subject.

In the context of the present invention, the term "subject", as used in certain embodiments, preferably refers to a mammal, such as a mouse, rat, guinea pig, rabbit, cat, dog, monkey, or preferably a human. The subject of the invention may be at danger of suffering from a disease, such as an infection, for example a bacterial infection, a viral infection, a fungal infection, or a parasitic infection. A more detailed description of medical indications relevant in context of the invention is provided herein elsewhere.

As used herein, the term "patient" refers to any animal (e.g., a mammal), including, but not limited to, humans, non-human primates, rodents, and the like, which is to be the recipient of a particular treatment or diagnostic method. The term "patient" preferably refers to a human, for example a human patient, for whom diagnosis, prognosis, or therapy is desired. Typically, the terms "subject" and "patient" are used interchangeably herein in reference to a human subject. As used herein, the term "subject suspected of having a disease" refers to a subject that presents one or more symptoms indicative of a disease. A subject suspected of having a disease may also have one or more risk factors. The present invention is also applicable as follow-up care for monitoring a subject for a recurrence of the disease.

In a particularly preferred embodiment, the subject is a mammal, such as a mouse, a rat, a guinea pig, a rabbit, a cat, a dog, a monkey, or a human, preferably a human, such as a human patient, more preferably a human patient suffering from a disease, such as a bacterial infection, and in need of a therapy, most preferably a human patient suffering from a bacterial infection and in need of a therapy.

In a further preferred embodiment, the subject is a mammal, such as a mouse, rat, guinea pig, rabbit, cat, dog, monkey, or preferably a human, such as a human patient, optionally wherein said compound for use is administered to the subject in a therapeutically effective amount.

Yet another aspect of this invention relates to A pharmaceutical composition, comprising: dicumarol, or a pharmaceutically acceptable salt thereof and at least one macrolide antibiotic, such as erythromycin, for use in treating dysbiosis, and in antagonizing the antibacterial effect of the at least one macrolide antibiotic, such as the erythromycin, in *Phocaeicola,* such as, for example, *P. vulgatus,* and wherein said dicumarol, or the pharmaceutically acceptable salt thereof, does not antagonize the antibacterial effect of said at least one macrolide antibiotic, such as erythromycin, in at least one second pathogenic bacterium, such as, for example, *S*. *aureus* or *S. pneumoniae.*

In the context of this invention, the term "pharmaceutical composition" shall refer to a preparation which is in such form as to permit the biological activity of an active ingredient contained therein to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. Pharmaceutically acceptable diluents include, for example, saline and aqueous buffer solutions. A "pharmaceutically acceptable carrier" refers to an ingredient in a pharmaceutical formulation, other than an active ingredient, which is nontoxic to a subject. Pharmaceutically acceptable carriers include any and all solvents, dispersion media, coatings, antibacterial and antifungal agents, isotonic and absorption delaying agents, and the like that are physiologically compatible. The carrier can be suitable for intravenous, intramuscular, subcutaneous, parenteral, spinal, epidermal, or any other commonly used route of administration (e.g. by injection or infusion).

The pharmaceutical compositions for use according to this invention may also contain adjuvants such as preservatives, wetting agents, emulsifying agents and dispersing agents. Prevention of presence of microorganisms may be ensured both by sterilization procedures, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents, which delay absorption, such as aluminum monostearate and gelatin.

The pharmaceutical compositions of this invention must be sterile and fluid to the extent that the composition is deliverable by syringe. In addition to water, in one embodiment the carrier is an isotonic buffered saline solution. Proper fluidity can be maintained, for example, by use of coating such as lecithin, by maintenance of required particle size in the case of dispersion and by use of surfactants. In many cases, it is preferable to include isotonic agents, for example, sugars, polyalcohols, such as mannitol or sorbitol, and sodium chloride in the composition.

The at least one second compound (ii) is selected from a macrolide, preferably wherein said antibiotic is selected from erythromycin.

The at least one first compound (i) for use or the pharmaceutically acceptable salt thereof of the pharmaceutical composition for use of this invention is Dicumarol, or a pharmaceutically acceptable salt thereof.

In the context of this invention Dicumarol shall refer to 3,3'-Methylene-bis(4-hydroxycoumarin).

Yet another preferred embodiment relates to the pharmaceutical composition for use of this invention, wherein said at least one first bacterium is member of the genus *Phocaeicola,* such as, for example, *P. vulgatus,*

Preferred is that said at least one first bacterium is at least one bacterial species selected from *Phocaeicola vulgatus, Phocaeicola coprocola, and Phocaeicola dorei.*

A further preferred embodiment relates to the pharmaceutical composition for use of this invention, wherein said at least one second bacterium is a Gram-positive bacterium or a Gram-negative bacterium, and wherein said at least one second bacterium is a member of a genus selected from *Enterococcus, Staphylococcus, Enterobacter, Streptococcus, Pseudomonas, Escherichia, Salmonella, Helicobacter, Neisseria, Campylobacter, Chlamydia, Clostridia, Citrobacter, Vibrio, Treponema, Mycobacterium, Klebsiella, Actinomyces, Bacteroides, Bordetella, Borrelia, Brucella, Corynebacteria, Diplococcus, Fusobacterium, Leptospira, Listeria, Pasteurella, Proteus, Rickettsia, Shigella, Yersinia, Parabacteroides, Odoribacter, Faecalibacteria, Collinsella, Eggerthella, Lactonifactor, Pediococcus, Leuconostoc, Lactococcus, Roseburia, Coliform, Bacillus, Franscicella, Acinetobacter, Legionella, Actinobacillus, Coxiella, Kingella kingae, Haemophilus,* and combinations thereof, optionally wherein said at least one second bacterium is an antibiotic-resistant bacterium and/or a multi-drug resistant bacterium.

In a further preferred embodiment, said at least one second bacterium is a pathogenic bacterium.

An additionally preferred embodiment pertains to the afore-described pharmaceutical composition for use according to this invention, wherein said components (i) and (ii) of said pharmaceutical composition are administered to a subject simultaneously, separately, or sequentially.

Yet another preferred embodiment relates to a pharmaceutical composition for use of this invention, wherein said pharmaceutical composition is in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, or room spray.

A further preferred embodiment relates to the pharmaceutical composition for use of this invention, wherein said pharmaceutical composition is administered to a subject. In a particularly preferred embodiment, said subject is a mammal, such as a mouse, rat, guinea pig, rabbit, cat, dog, monkey, or preferably a human, such as a human patient, optionally wherein said pharmaceutical composition for use is administered to said subject in a therapeutically effective amount, thereby preventing and/or treating a disease in said subject, and/or preventing a modification of the microbiome of said subject.

Yet another preferred embodiment relates to the compound for use of this invention, wherein said compound for use is administered to the subject by any suitable means including oral, intravenous, arterial infusion, intraperitoneal, parenteral, enteral, topical, intramuscular, subcutaneous, surgical, topical, cutaneous, subcutaneous, or any other clinically approved way of administration.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection, and infusion. Regardless of the route of administration selected, the compounds and pharmaceutical compositions of the present invention, which may be used in a suitable hydrated form, are formulated into pharmaceutically acceptable dosage forms by conventional methods known to those skilled in the art. Actual dosage levels of the active ingredients in the pharmaceutical compositions of the present invention may be varied so as to obtain an amount of the active ingredient, which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and other factors well known in the medical arts.

The dosage regimen of the compounds for use of this invention and/or the pharmaceutical composition for use of this invention will be determined by the attending physician and clinical factors. As is well known in the medical arts, dosages for any one patient depend upon many factors, including the patient's size, body surface area, age, the particular compound to be administered, sex, time and route of administration, general health, and other drugs or compounds being administered concurrently. A typical dose can be, for example, in the range of 0.001 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment.

The skilled artisan will understand that numerous methods may be used to determine the dosage regimen of the compounds for use of this invention and/or the pharmaceutical compositions for use.

The compounds for use and pharmaceutical compositions for use of the invention may be administered locally or systemically. Administration will preferably be parenterally; the compounds and pharmaceutical compositions may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils, such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present, such as, for example, antimicrobials, anti-oxidants, chelating agents, and inert gases and the like.

Furthermore, the compounds for use and pharmaceutical compositions for use of the invention may comprise further agents such as interleukins or interferons depending on the intended use of the compounds and pharmaceutical compositions.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B, and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The figures show:

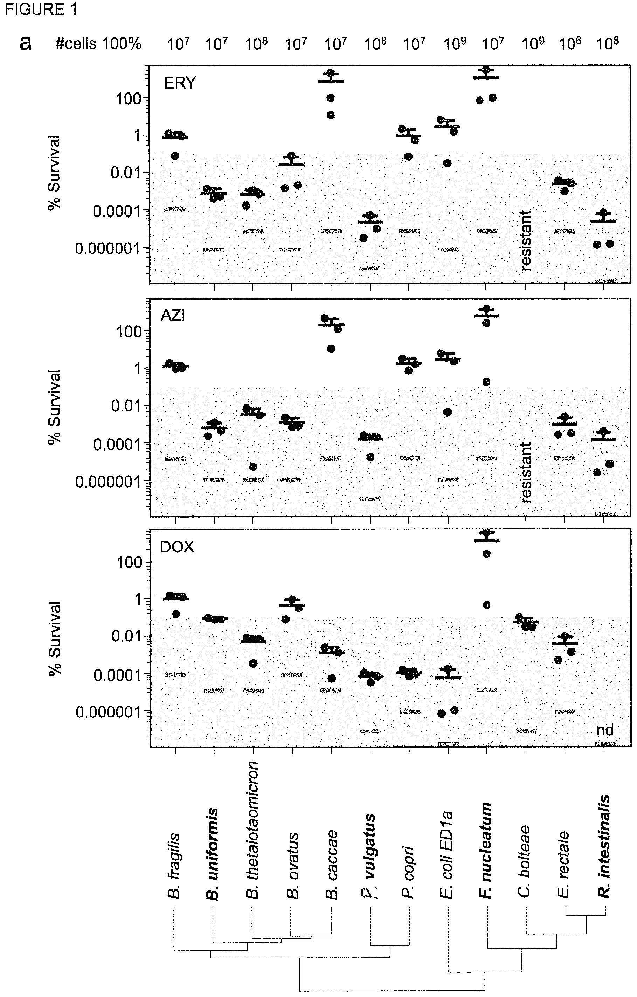

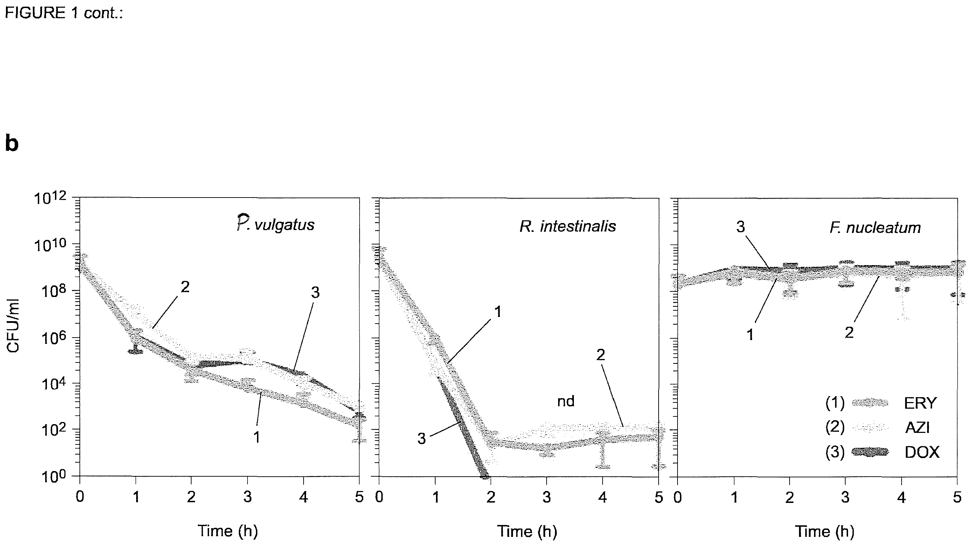

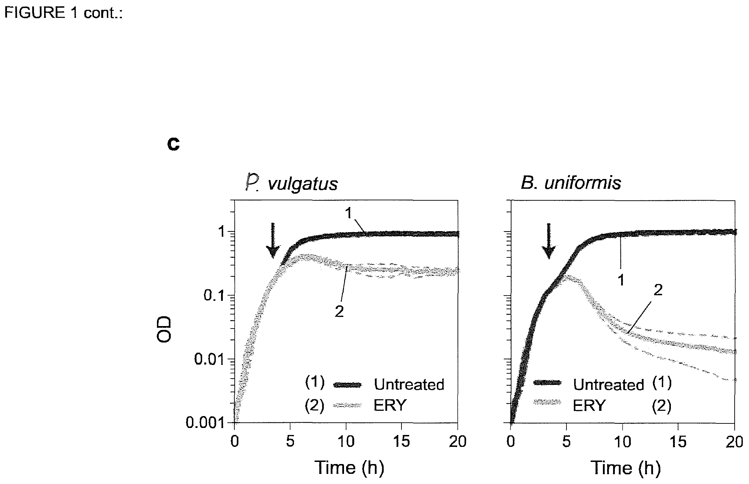

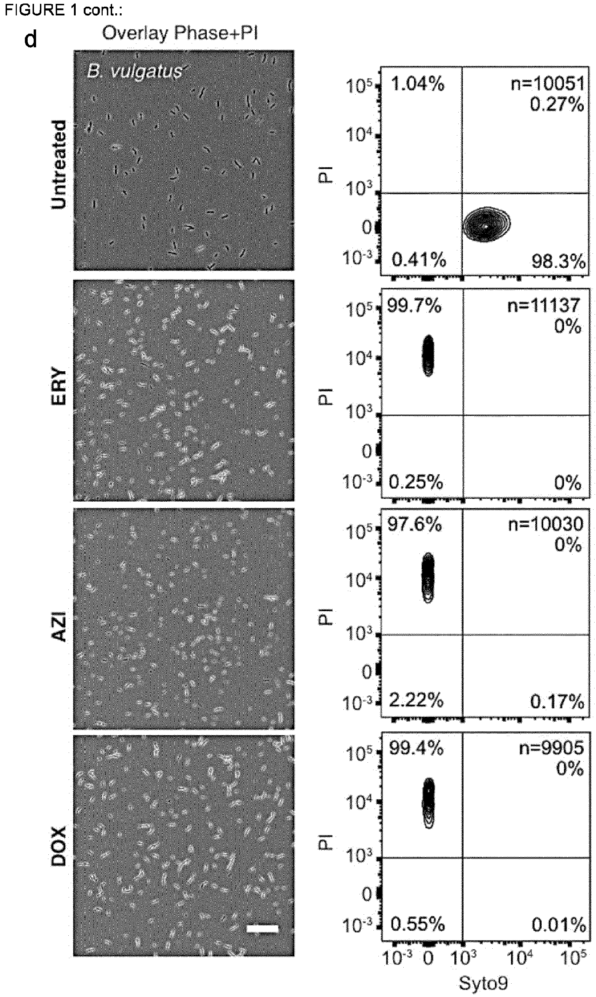

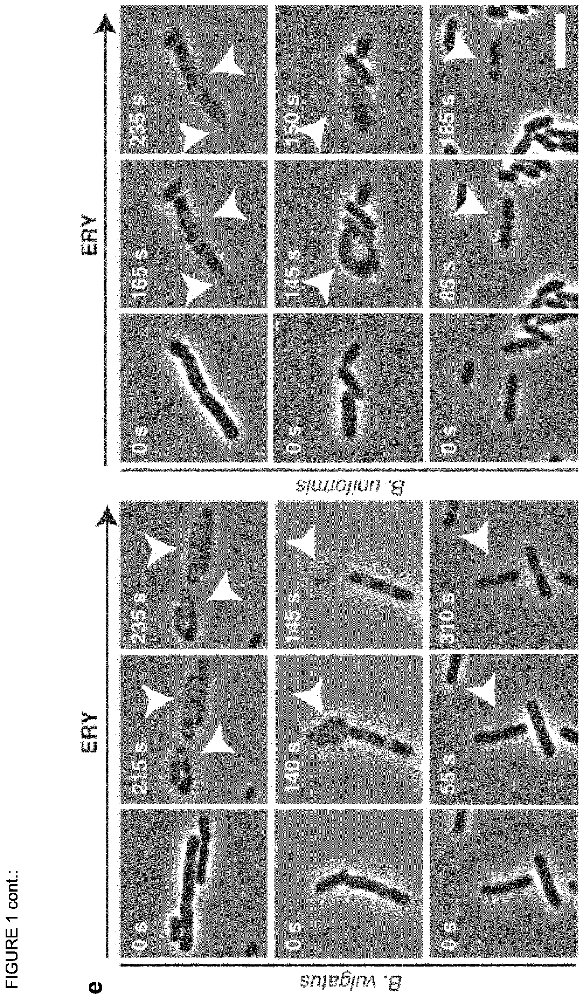

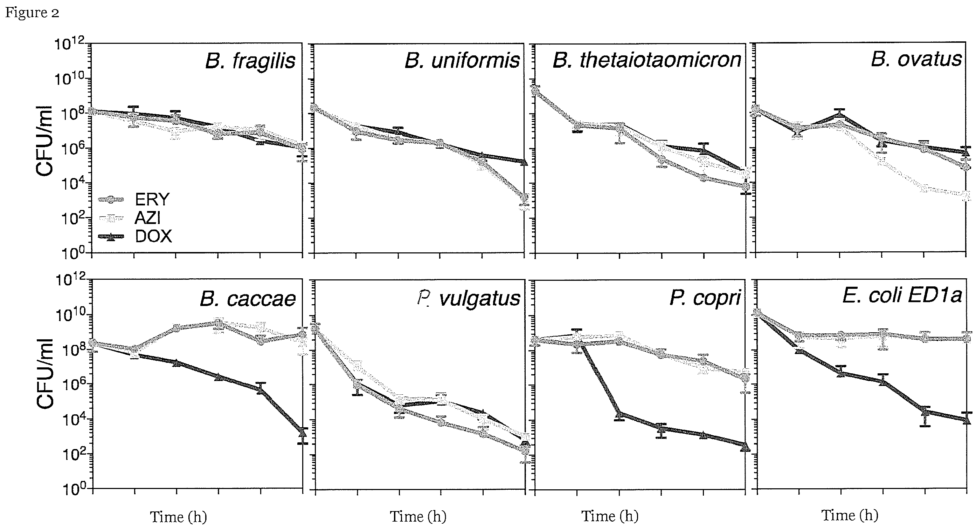

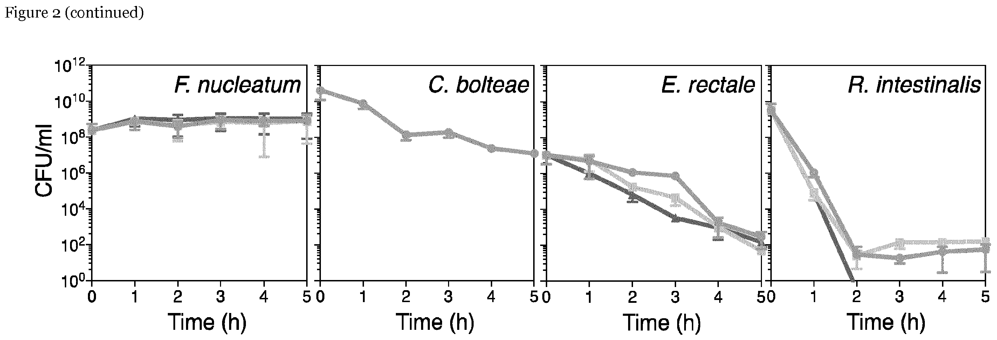

**Figure 1** **shows macrolides and tetracyclines kill human gut commensal species. a.** The survival of 12 abundant gut microbe species was measured after a 5-hour treatment with a 5-fold MIC of erythromycin (ERY), azithromycin (AZI) or doxycycline (DOX). The survival was assessed by counting CFUs/ml before and after antibiotic treatment. The number of CFUs/ml before treatment was set as 100%. The detection limit for each experiment (gray bar) and the bactericidal threshold (shaded area) are indicated. Species are plotted according to phylogeny (IQTree, Methods) and in bold are noted the species that are used in later panels. The graph shows the mean+SD of 3 independent experiments. **b.** Time-kill curves of *P. vulgatus, R. intestinalis* and *F. nucleatum* after antibiotic treatments. Survival was assessed by CFU counting over a 5 hour-treatment of ERY, AZI or DOX. This graph shows the mean±SD of 3 independent experiments. Nd: non-detectable. Time-kill curves for the other tested gut microbes can be found in Fig. 2. **c.** Erythromycin induces lysis of *P. vulgatus* and *B. uniformis. P. vulgatus* and *B. uniformis* were grown for 3 hours before addition (yellow) or not (black) of 15 µg/ml ERY treatment (5-fold MIC; yellow) as indicated by the arrow. Growth curves were acquired for 20 hours. This graph shows the mean±SD (dotted line) of 3 independent experiments. **d.** Live/dead staining of macrolide or tetracycline-treated *P*. *vulgatus.* The left panel shows an overlay of phase contrast and fluorescence microscopy images of propidium iodide (PI)-stained *P. vulgatus* before and 5 hrs after ERY, AZI or DOX treatment. Cultures were concentrated before imaging; the scale bar is 10 µm. The right panel shows the corresponding quantification of live/dead-stained cells by flow cytometry with Syto9 on the x-axis (live cells) and PI on the y-axis (dead cells). Both the total number of measured events (n) and the percentage of cells found in each quadrant are indicated. **e.** Erythromycin induces blebbing, cytoplasmic shrinkage and lysis in *P. vulgatus* and *B. uniformis.* Phase contrast movies of *P. vulgatus* and *B. uniformis* were acquired after ERY treatment (5-fold MIC). Here shown are 3 frames of 3 images per strain (time indicated in the upper left corner; t=0 when drug added). White arrows indicate blebs, cytoplasmic shrinkage and bacterial lysis; the scale bar is 5 µm.
**Figure 2** **shows time-kill curves of 12 abundant gut microbes after treatment with macrolides and tetracyclines.** Survival of 12 abundant gut microbes was assessed by CFU counting over a 5 hour-treatment of either ERY, AZI or DOX. This graph shows the mean±SD of 3 independent experiments.

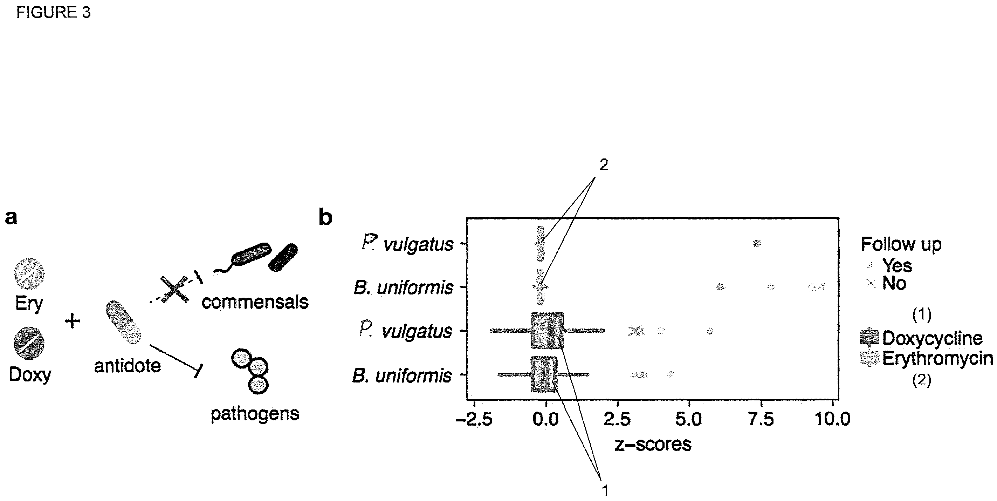

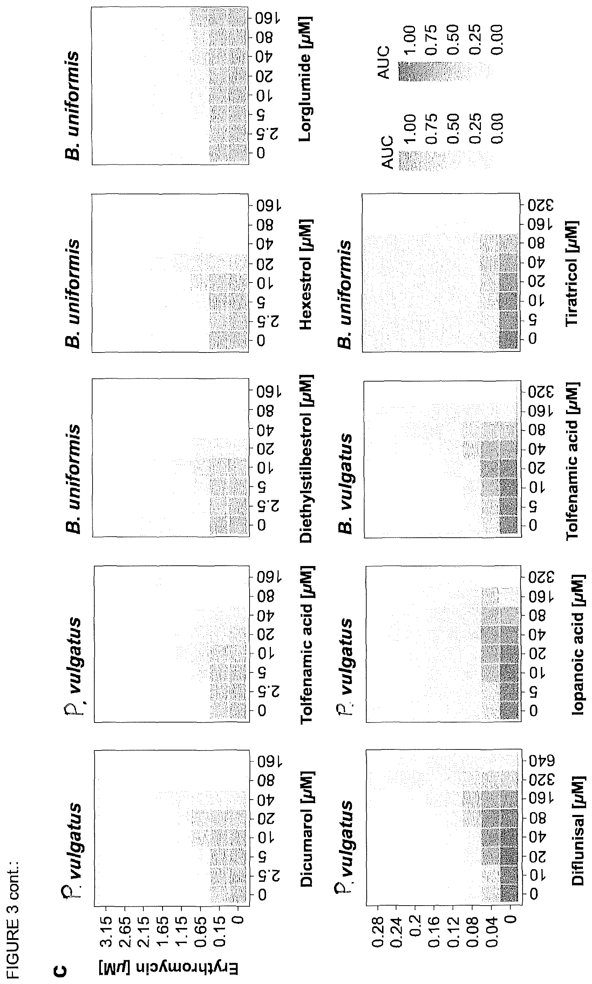

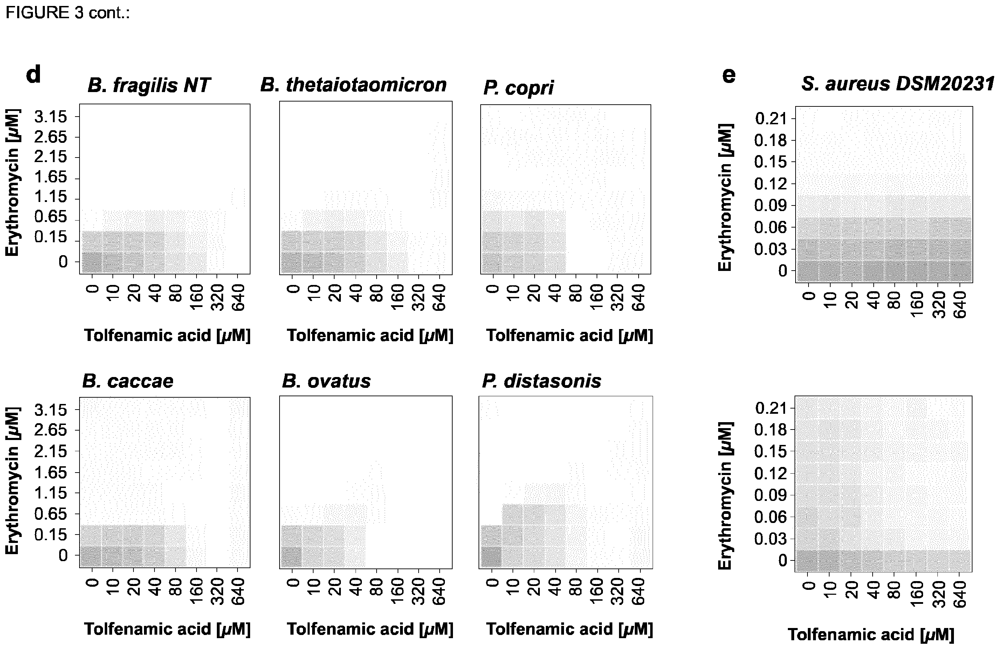

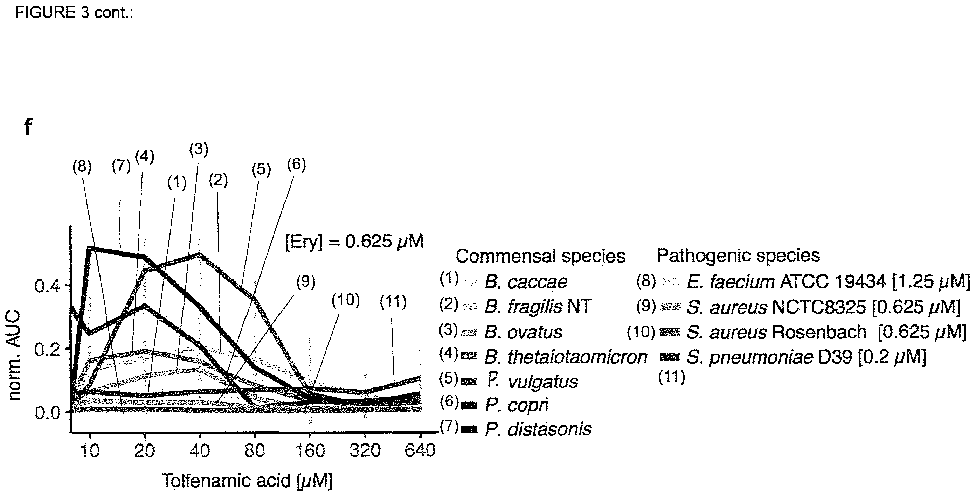

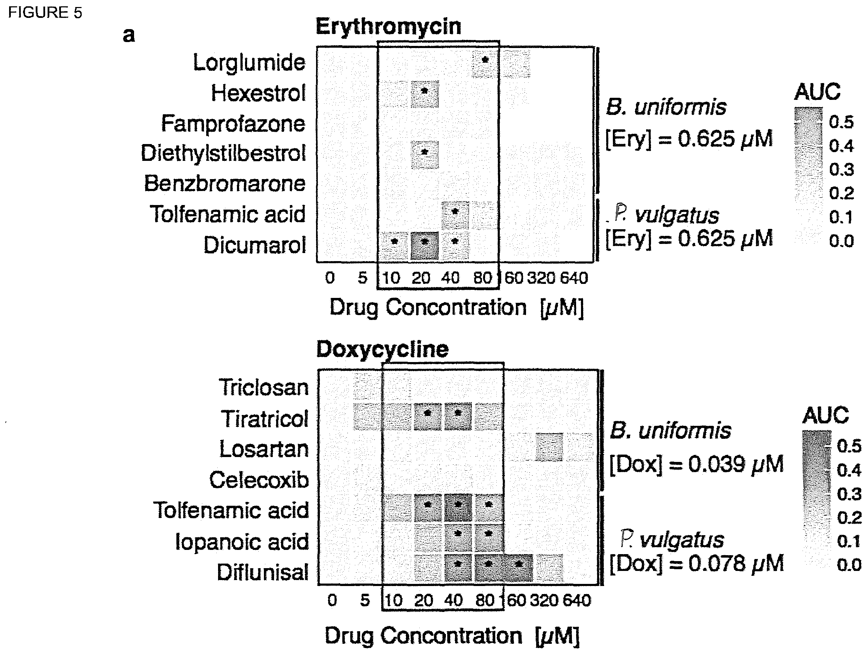

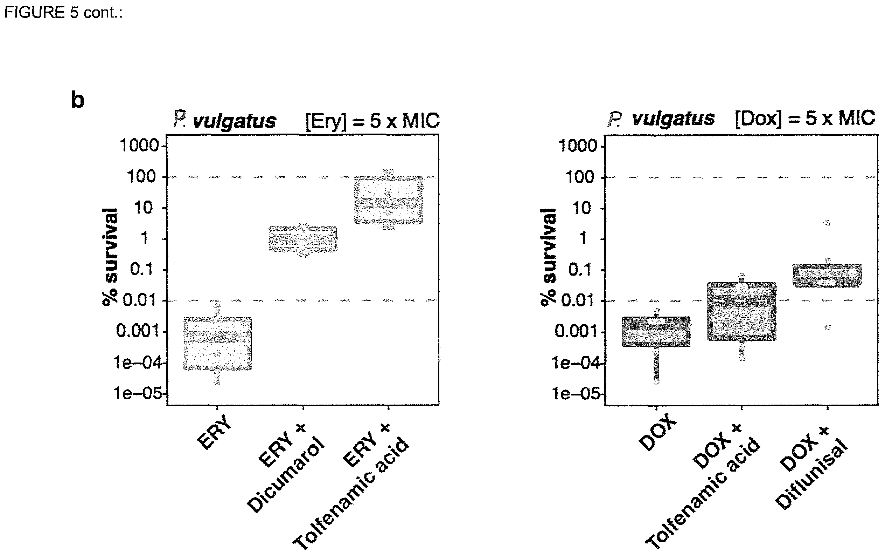

**Figure 3** **shows antidotes for selective protection of prevalent and abundant gut commensal species from macrolides and tetracyclines. a.** Schematic illustration of the screen concept: searching for antidote compounds that antagonize the antibacterial effect of erythromycin or doxycycline on commensal but not on pathogenic bacteria. **b.** Z-scores on bacterial growth (based on areas under the curve (AUCs)) for combinatorial drug exposure with antibiotic (ERY or DOX) and FDA-approved drug. Compounds that successfully rescued *P*. *vulgatus* and/or *B. uniformis* growth in the presence of the antibiotic (z-score > 3) are indicated in gray. The strongest hits (circles) were validated further in concentration-dependent assays (Fig. 5 a). For each antibiotic and each strain, ~1200 drugs were tested in two replicates. Boxplots are plotted as in Fig. 1 d. **c.** For 9 of the validated antagonists, 8 x 8 checkerboard assays were performed to determine concentration ranges of the antagonistic interaction. Heat maps depict bacterial growth based on normalized median of AUCs of 4 replicates. Antagonistic interactions are framed in red, and pairs tested further in other commensal species in bold. **d.** Checkerboard assays confirm the ability of tolfenamic acid to protect further gut commensals from growth inhibition by erythromycin. Heat map as in **c,** but for 2 replicates. Antagonistic interactions are framed in red. **e.** Checkerboard of tolfenamic acid with erythromycin reveal neutral interactions in *S. aureus* and *E. faecium* (aerobic conditions). Heat maps as in c. **f.** Tolfenamic acid concentration-dependent rescue of commensal growth at clinically relevant erythromycin concentrations based on AUCs (anaerobic conditions). Erythromycin still retains its activity against pertinent pathogens such as *S. aureus* and *S. pneumoniae* (aerobic conditions). 0.625 µM correspond to ~0.5 µg/ml erythromycin, which is in the range of the MIC breakpoints for *Staphylococcus* (1 µg/ml), *S. pneumoniae* (0.25 µg/ml) and *Streptococcus* groups A, B, C & G (0.25 µg/ml). Error bars depict standard deviation.

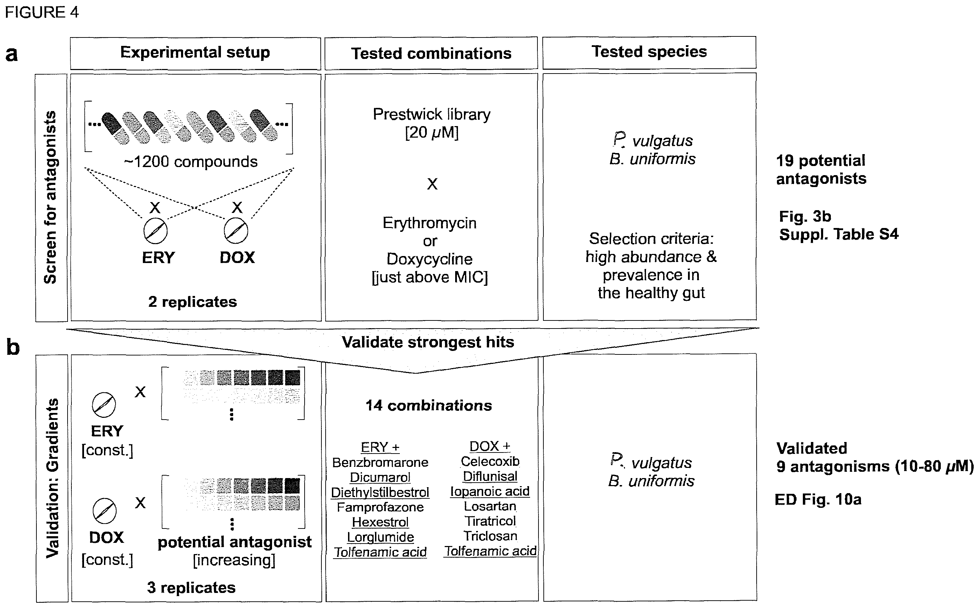

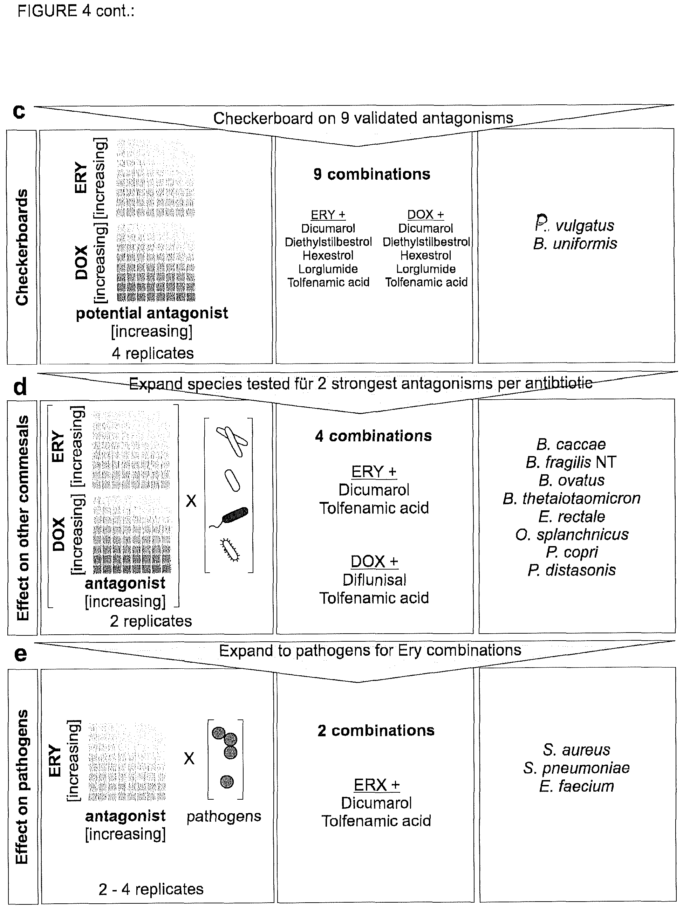

**Figure 4** **shows a schematic overview of the screen for microbiome-protective antibiotic antagonisms.** Workflow with decision process which antagonist to move on to next evaluation step.
**Figure 5** **shows validation of potential microbiome-protective antagonists. .a.** Validation of the strongest antagonists in independent experiments. Erythromycin and doxycycline concentrations were kept constant ([ERY]=0.625 µM, [DOX] = 0.039 / 0.078 µM) and concentration ranges were tested for antagonist. Asterisks indicate that at least 25% of the bacterial growth (compared to no drug controls) could be rescued by the antagonist at a given concentration. Heat map depicts median AUCs across triplicates. **b.** Percentage of surviving *P*. *vulgatus* cells were determined after 5 h incubation with either erythromycin (3.25 µM) or doxycycline (0.4 µM) alone or in presence of the antagonist dicumarol (20 µM), tolfenamic acid (40 µM) or diflunisal (80 µM). Data is based on 3 replicates. Boxplots are plotted as in Fig. 1 d.

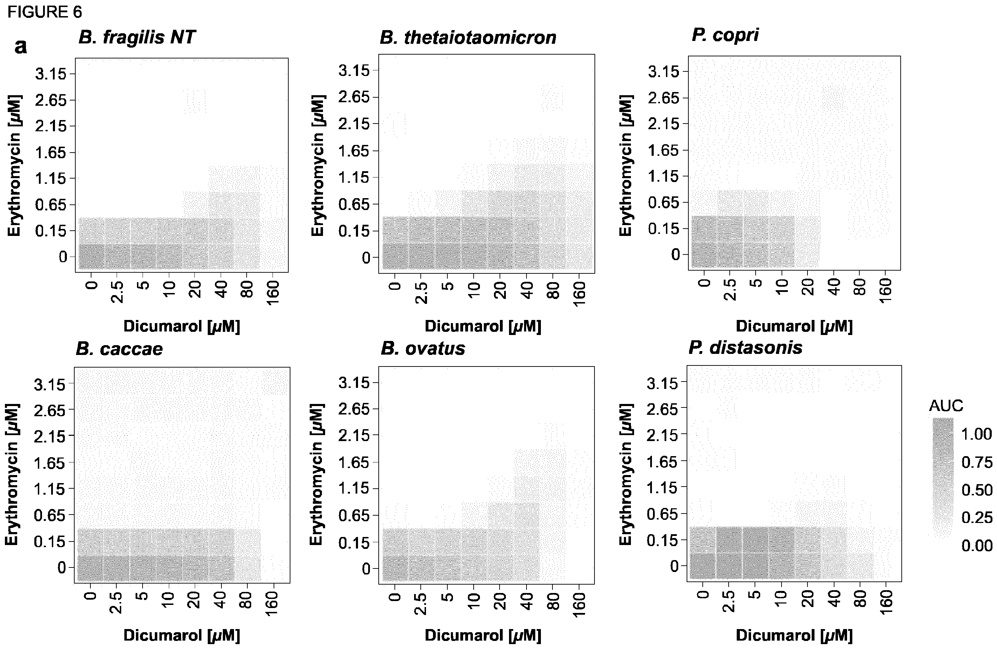

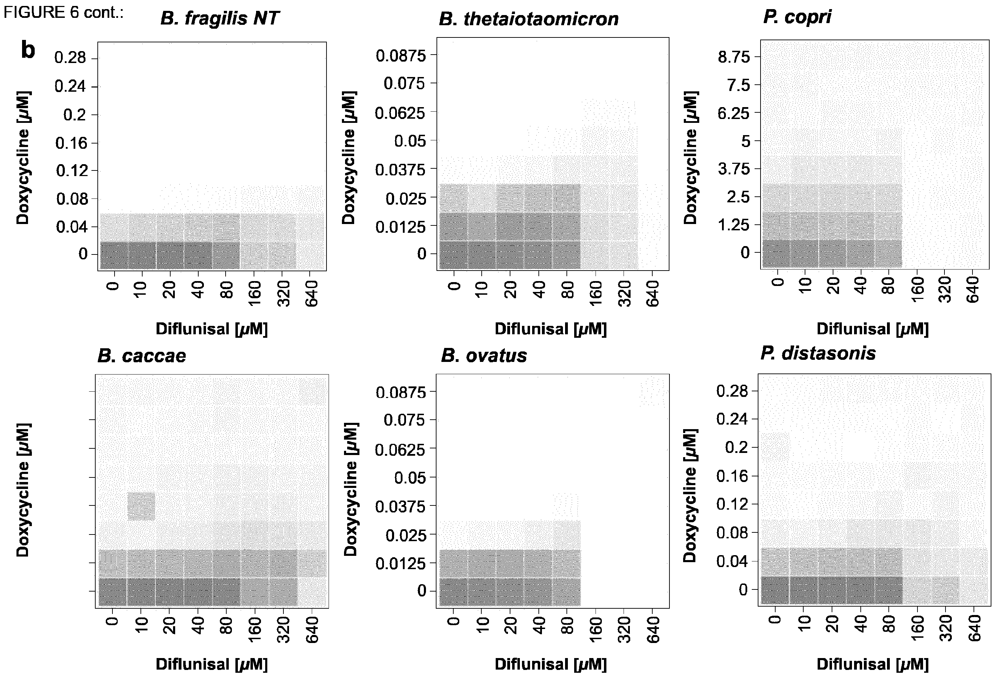

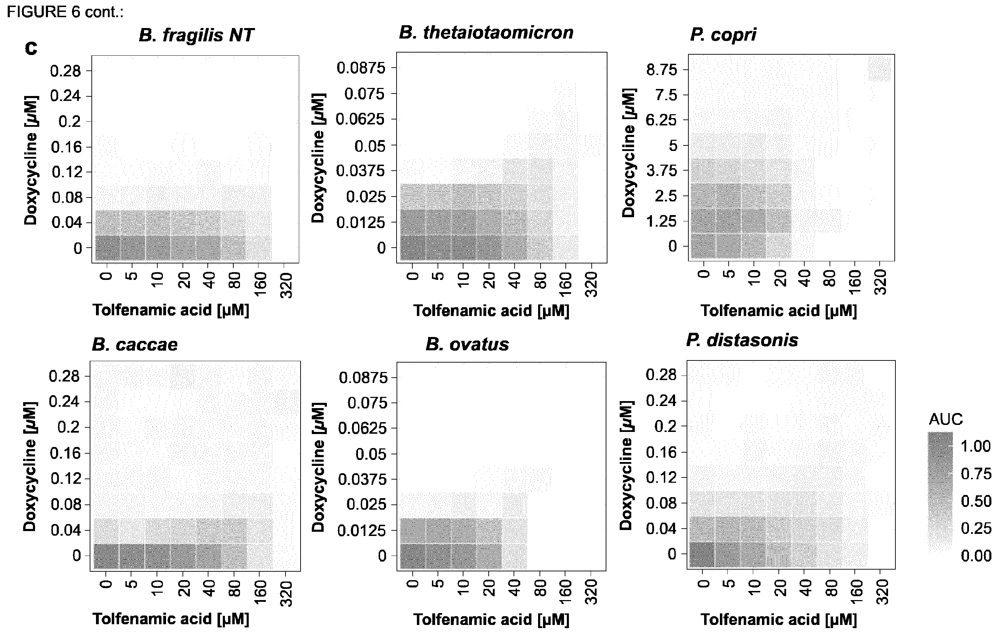

**Figure 6** **shows the effect of antidotes on further gut commensals.** 8 x 8 checkerboard assays to investigate if antidote is also protective for additional gut commensals for the following combinations: erythromycin and dicumarol (**a**), doxycycline and diflunisal (**b**) and doxycycline and tolfenamic acid (**c**). Heat map depicts bacterial growth based on median AUCs from two independent replicates. Red contours indicate antagonistic drug interactions.

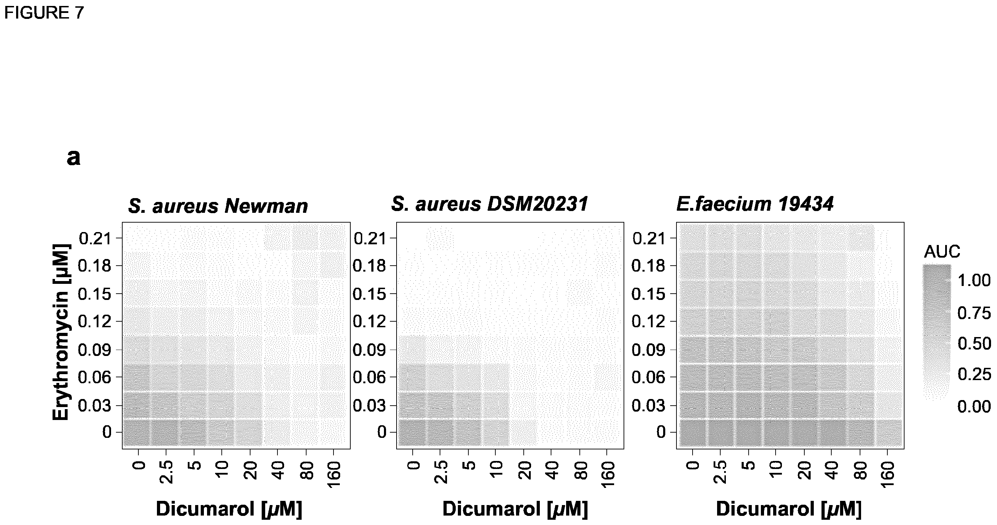

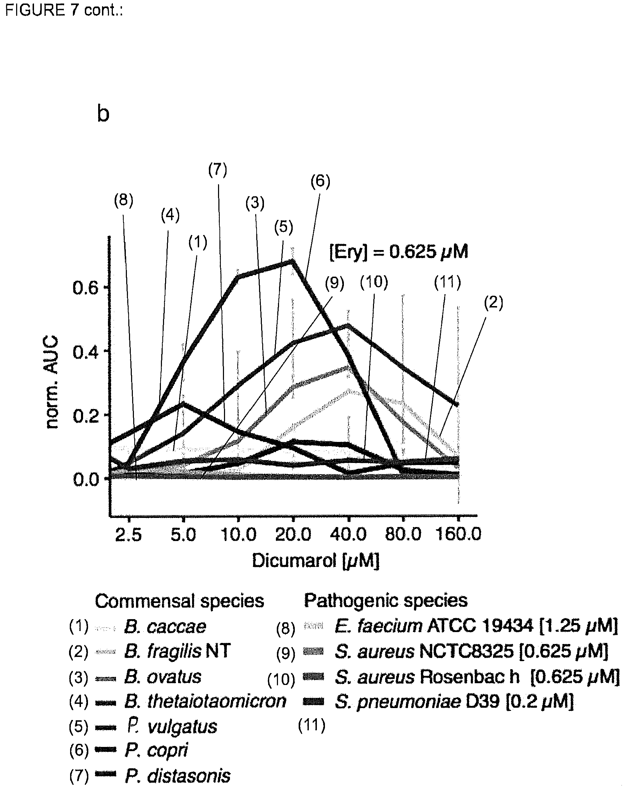

**Figure 7** **shows the effect of the antidote dicumarol on pathogens, relatively to commensal species. a.** Checkerboard assays for the drug combinations erythromycintolfenamic acid and erythromycin-dicumarol on the pathogens *S. aureus* (two different strains) and *E. faecium.* Heat map depict median normalized AUCs of checkerboard assays (at least three independent replicates). **b.** Dicumarol rescues commensal growth (based on median AUCs, N=2) at clinically relevant erythromycin concentrations in a concentration-dependent manner. Erythromycin still retains its activity against pertinent pathogens such as *S. aureus* and *S. pneumoniae* (based on median AUCs, N=3). Error bars depict standard deviation.

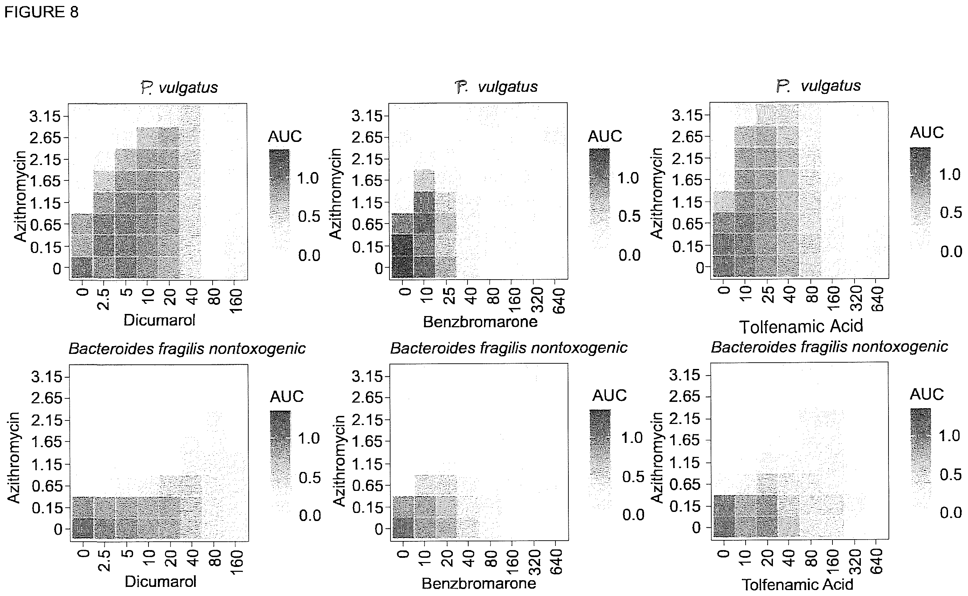

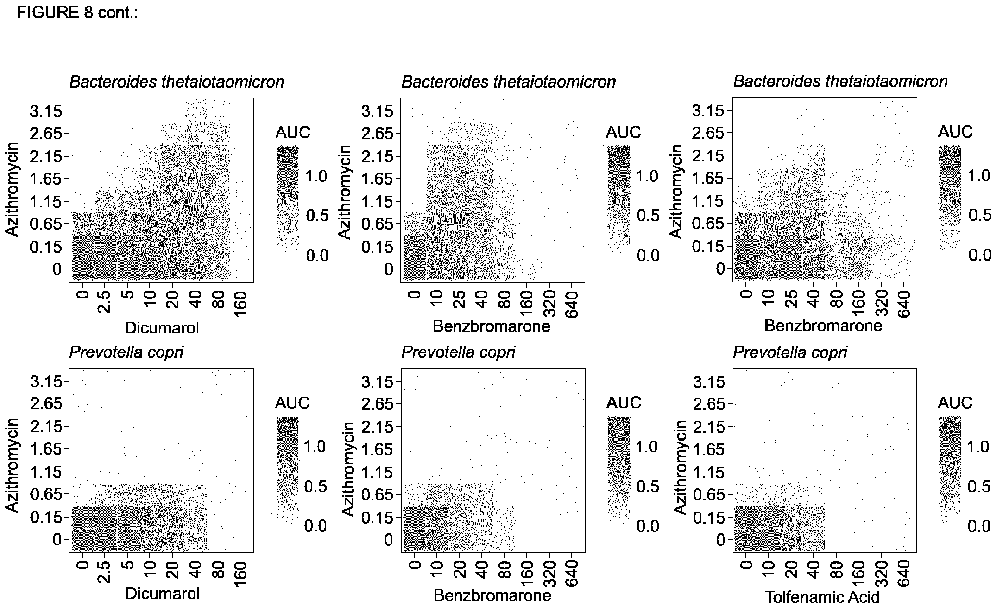

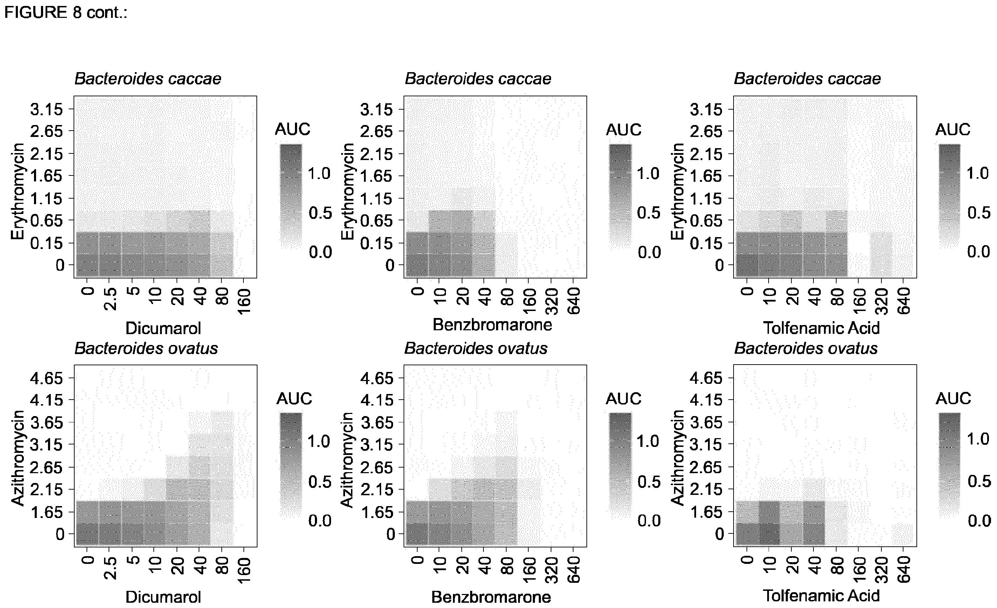

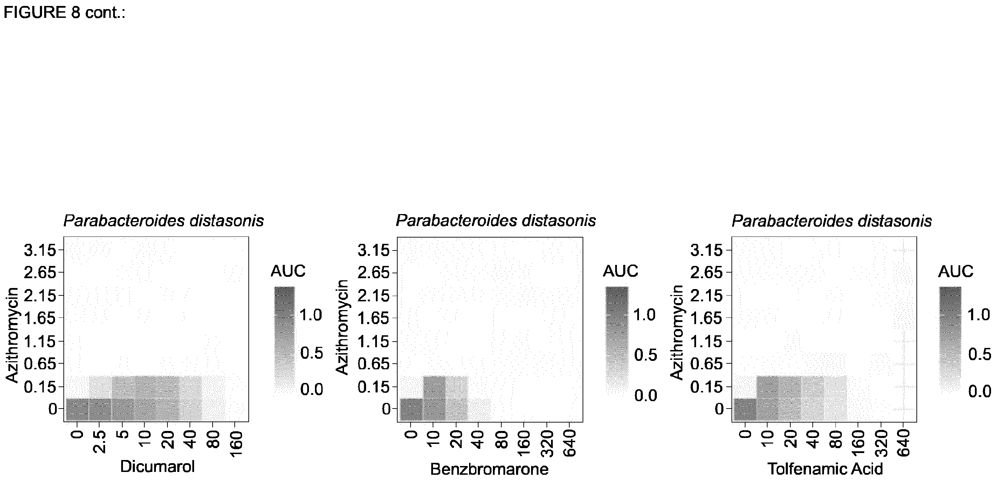

**Figure 8** shows the effect of the antidote dicumarol, benzbromarone or tolfenamic acid on azithromycin treatment of species of the order bacteroidales (Example 4). The antidotes dicumarol (first column), benzbromarone (second column) and tolfenamic acid (third column) were tested for their efficacy in antagonizing the macrolide antibiotic azithromycin across seven prevalent and abundant gut *Bacteroidales* and *Phocaeicola* (P. vulgatus, B. fragilis NT, B. thetaiotaomicron, P. copri, B. caccae, B. ovatus, P. distasonis). Heat maps depict bacterial growth based on normalized median of the area under the growth curve (AUCs) of 3 replicates. All drug concentrations are in µM.

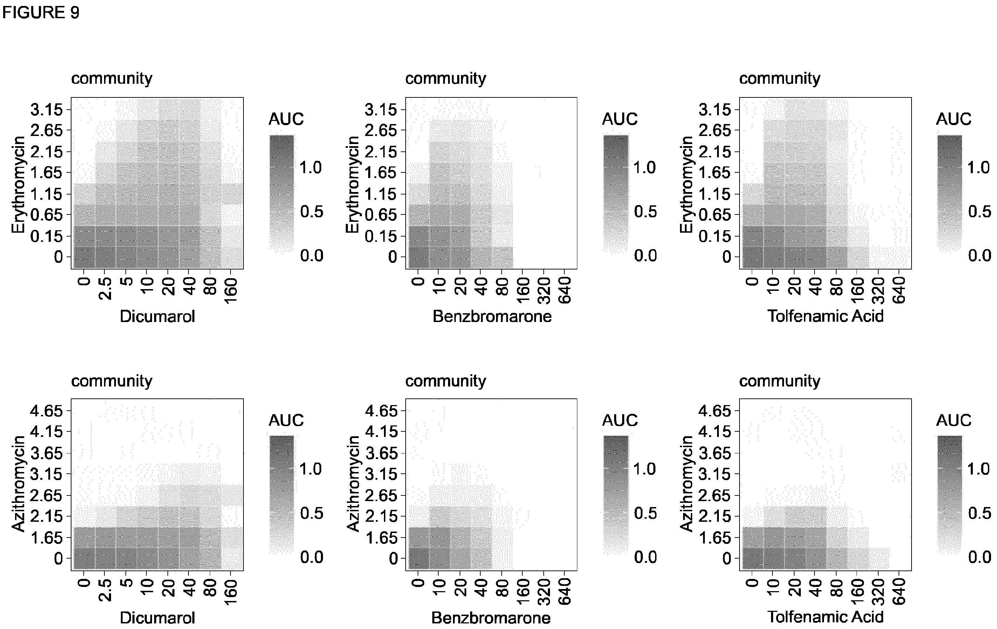

**Figure** 9 shows that synthetic gut commensal communities can be protected from macrolide antibiotics by the antidotes dicumarol, benzbromarone or tolfenamic acid (Example 5). Heat maps depict area under the growth curve (AUC) of a synthetic community consisting of seven human gut commensals. Shown is the median AUC of three individual replicates. All drug concentrations are in µM.

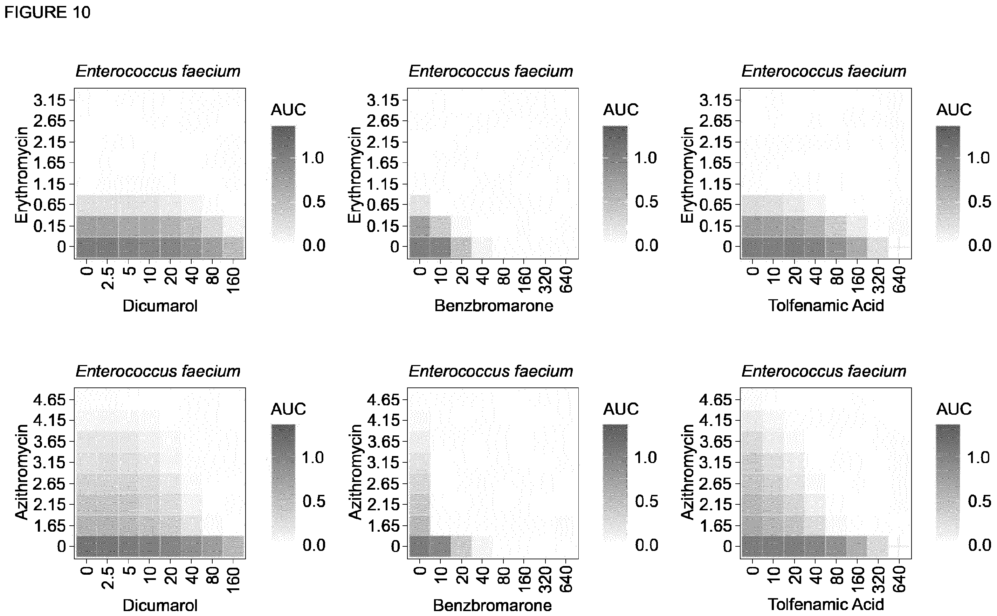

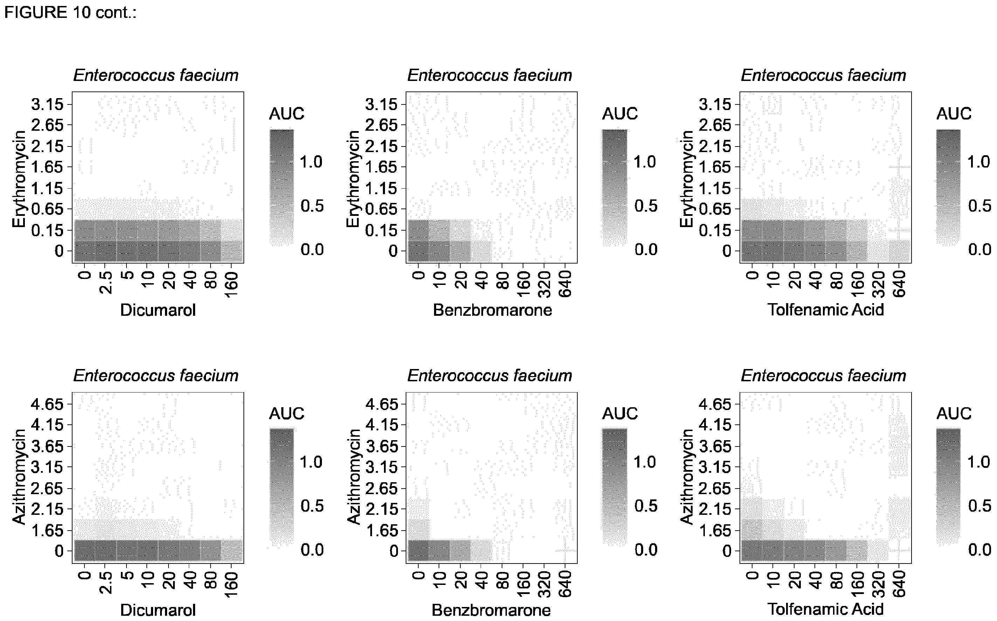

**Figure 10** shows that human intestinal pathogens of the genus enterococcus are not rescued from macrolide antibiotics by the antidotes dicumarol, benzbromarone or tolfenamic acid (Example 6). Heat maps show median area under the growth curve (AUC) of three biologically independent replicates. Benzbromarone and tolfenamic acid show synergistic effects with azithromycin/erythromycin on E. faecalis/E. faecium. All drug concentrations are in µM.

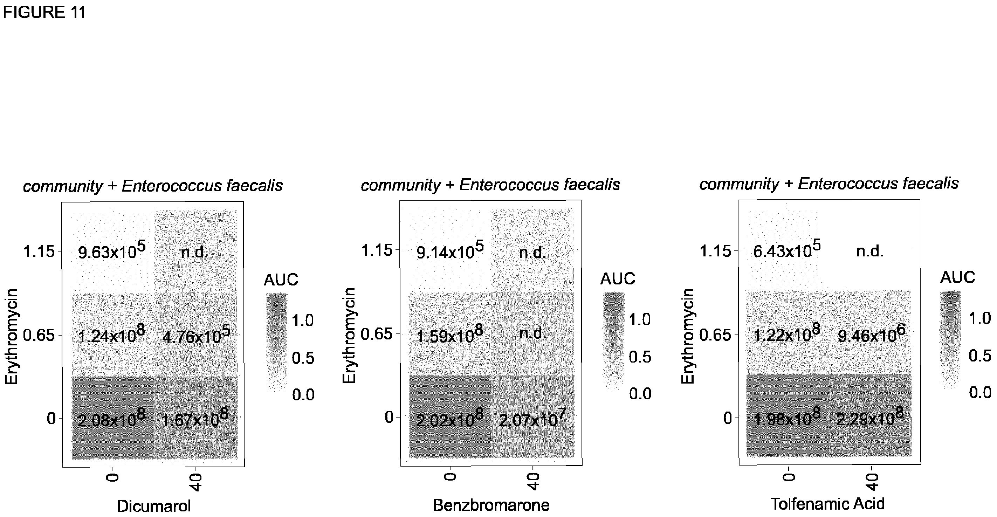

**Figure 11** shows that the antidotes dicumarol and benzbromarone selectively rescue growth of commensal bacteria from erythromycin treatment, while pathogenic enterococci are inhibited (Example 7). Heat maps show median area under the growth curve (AUC) of the seven-member synthetic community including the pathogen E. faecalis. The numbers within the heat map represent the total number of all E. faecalis viable cells for each erythromycin-antidote combination as determined by enumerating of E. faecalis colony forming units after plating on selective medium. All drug concentrations are in µM. n. d. = not detectable.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

**Growth conditions:** All experiments from this study were performed in an anaerobic chamber (Coy Laboratory Products Inc) (2% H₂, 12% CO₂, 86% N₂) and all materials and solutions used for these experiments were pre-reduced for at least 24 h before use unless specified otherwise. Bacteria used in this study were typically pre-cultured for 2 overnights: Cells were cultured in 5 ml modified Gifu Anaerobic Medium broth (MGAM) (HyServe GmbH & Co.KG, Germany, produced by Nissui Pharmaceuticals) and grown at 37°C overnight. The next day, cells were diluted 1/100 in 5 ml MGAM medium and grown at 37°C for a second overnight before starting the experiments.

**Quantitative assay for minimum inhibitory concentration determination with MICs test strips:** MICs test strips were purchased from Liofilchem or Oxoid. All MICs were measured under anaerobic growth conditions inside a Coy anaerobic chamber. Bacteria were precultured in MGAM for two overnights and cultures were diluted to OD₅₇₈ = 0.5. 50 µl of the diluted culture were spread on a MGAM agar plate and allowed to dry for 15 min. The MIC test strip was placed on the agar with sterile tweezers, allowing the part with the lowest concentration touch the agar first. Plates were incubated at 37°C inside the anaerobic chamber, at least overnight and longer depending on the species-specific growth requirements. After formation of a symmetrical inhibition ellipse, plates were taken out of the chamber and imaged under controlled lighting conditions (spImager S&P Robotics Inc.) using a 18 megapixel Canon Rebel T3i (Canon Inc. USA). MICs are directly determined from the strip scale at the point where the edge of the inhibition ellipse intersects the MIC test strip. All MICs were determined in duplicates. In cases of an eight-fold difference between the two values, a third replicate was done. In all cases, this resulted in a clear outlier (> 8-fold different from other two MICs) that was removed from the dataset.

**MIC comparison to ChEMBL and EUCAST databases:** Previously known MICs were extracted from the ChEMBL database (version 24) and EUCAST (obtained on May 14, 2018). Antibiotics from these two datasets were mapped to the inventor's dataset by name. Species were mapped using NCBI Taxonomy Identifiers and species names. For MICs from ChEMBL, a keyword-based approach was used to exclude experiments on species with mutations, deletions, insertions, etc. The EUCAST database contains a large number of reported MICs for each compound-species pair. The inventors collapsed these to a single value by calculating the median MIC.

Estimates on the abundance and prevalence of species in the healthy human gut microbiome were calculated using mOTUs v2 as follows: Relative species abundances were determined in 727 shotgun metagenomic samples from donors in the control groups of multiple studies from various countries and continents. Prior to taxonomic profiling, metagenomes were quality controlled using the MOCAT2-rtf procedure, which removed reads with ≥95% sequence identity and an alignment length of ≥45bp to the human genome hg19. Taxonomic profiles were then created using mOTUs version 2.1.0 with parameters -l 75 ; -g 2; and -c. Afterwards relative abundances below 10⁻⁴ were set to zero and species with nonzero abundance in <5 samples discarded. For the retained 1,350 species, prevalence was defined as the percentage of samples with nonzero abundance; a prevalence cut-off of 1% was chosen to classify species into "rare" and "common" species. For all species in the MIC dataset, the inventors manually assessed their status as pathogenic or non-pathogenic species using encyclopaedic and literature knowledge. Pathogenic species that occur in more than 1% of healthy people (i.e. are designated as "common") were classified as "potentially pathogenic species" that can, for example, cause diseases in immunocompromised patients.

**Killing curves and survival assay:** Cells were precultured as described in the *growth conditions* section before being diluted to an OD₅₇₈=0.01 and grown for 2 h at 37°C (unless specified otherwise). Next, cells were diluted 1/2 in MGAM containing a 10-fold MIC of erythromycin, azithromycin or doxycycline (final antibiotic concentration is 5-fold MIC) and incubated in the presence of the antibiotic for 5 h at 37°C. At several time-points (0, 1h, 2h, 3h, 4h, 5h), 100 µl of cells were serial-diluted in PBS (10⁻¹ to 10⁻⁸ dilutions) and plated on MGAM-Agar plates for colony forming unit (CFU) counting. When no cells were detected using this method, a bigger volume of culture (up to 2 ml) was plated to be able to detect CFUs. Agar plates were incubated overnight at 37°C and colonies were counted the next day, either manually, for low CFU numbers, or using the *Analyze Particles* tool from ImageJ³⁵,

**Live/dead staining:** Cells were precultured as described in the *growth conditions* section before being diluted to an OD₅₇₈=0.01 and grown for 2 h at 37°C. Cells were next diluted 1/2 in MGAM containing 10-fold MIC of erythromycin, azithromycin or doxycycline (final concentration is 5-fold the MIC) and incubated in the presence of the antibiotic for 5 hours at 37°C. Then, cells were live/dead stained using the *LIVE*/*DEAD BacLight Bacterial viability and counting kit* (#L34856 Molecular Probes, ThermoFisher) according to the manufacturer's protocol before and after antibiotic treatment.

**Flow cytometry:** Stained cells were counted using a BD LSRFortessa^{™} flow cytometer. The forward and side scatter signals (488 nm) as well as the green and red fluorescent signals (488-530/30A filter and 561-610/20A filter, respectively) were acquired. The FSC/SSC detectors were set to logarithmic scale. The flow rate varied between 12 µl/min and 60 µl/min depending on the concentration of each sample, and the analysis was stopped when 10,000 target events were measured. Graphs were generated using the FlowJo V10.3 software (Treestar).

**Microscopy:** For live/dead imaging, stained cells were washed twice in 0.85% NaCl before being spotted on 0.85% NaCl + 1% agarose pads between a glass slide and a coverslip. For time-lapse imaging, cells were precultured as described in the *growth conditions* section. Cells were then diluted to an OD₅₇₈=0.01 and grown for 3 h at 37°C before being spotted on MGAM +1% agarose pads, supplemented or not with 15 µg/ml erythromycin (5-fold MIC) between a glass slide and a coverslip. Slides were sealed with valap and taken outside of the chamber for imaging. The imaging was performed using a Nikon Eclipse Ti inverted microscope, equipped with a Nikon DS-Qi2 camera, a Nikon Plan Apo Lambda 60X oil Ph3 DM phase contrast objective and a Nikon HC mCherry filter set (Ex 562/40; DM 593; BA 641/75) to detect propidium iodide fluorescence. Images were acquired with the NIS-Elements AR4.50.00 software and processed with Fiji v.2.0.0-rc-68/1.52h.

**Growth curves:** Cells were precultured as described in the *growth conditions* section. Then, cells were diluted to an OD₅₇₈=0.01 in a 96-well plate sealed with a breathable membrane (Breathe-Easy^{®}) and grown for 2 h. Next, erythromycin was added to the culture to a final concentration of 15 µg/ml (5-fold MIC) and growth curves were acquired for 20 h using a microplate spectrophotometer (EON, Biotek) by measuring the OD₅₇₈ every hour after 30 sec of linear shaking.

The examples show:

### Example 1: Antibacterial effects on gut microbiota

The standard way to determine whether an antibiotic has bactericidal or bacteriostatic activity is to calculate time-kill curves, where the bacterial survivors are counted on agar at various time-points after drug treatment. If, over a significant period of antibiotic treatment (ranging from a 5 to 24 hours), the number of colony forming units (CFU)/ml of culture decreases by more than 99.9%, the antibiotic is considered bactericidal.

The inventors assessed the survival of 12 abundant gut microbes over a 5-hour treatment of either a macrolide (erythromycin or azithromycin) or a tetracycline (doxycycline) at 5 x MIC (Fig 1 a + b, Fig. 2).

Surprisingly, about half of the tested species decreased in survival by >99.9%, confirming that these drugs are bactericidal to several abundant gut microbes. To confirm this further, the inventors tested the viability of *P. vulgatus* and *E. coli* ED1a upon erythromycin, azithromycin or doxycycline treatments using live/dead staining. Microscopy and flow cytometry assessment of live/dead bacteria corroborated the initial observations (Fig. 1 c). As tetracylines are considered bona-fide bacteriostatic drugs in *E. coli,* the inventors were surprised to see that doxycycline effectively killed the commensal *E. coli* ED1a (Fig. 1 a).

The inventors also noticed that *P. vulgatus* and *B. uniformis* cultures decreased density in the presence of erythromycin (Fig. 1 d). The inventors confirmed by time-lapse microscopy that this was due to lysis. Erythromycin caused cell shape defects, including blebbing, cytoplasmic shrinkage, and ultimately cell lysis in both *P*. *vulgatus* and *B. uniformis* (Fig. 1 e). Altogether, the differential bactericidal activity of macrolides and tetracyclines on gut commensals could provide an explanation for the strong effects these drug classes have on the gut microbiota composition of human individuals.

### Example 2: Screen for microbiome-protective antibiotic antagonism

The inventors reasoned that it should be possible to identify drugs that selectively antagonize the effect of antibiotics on gut microbes, while retaining activity against pathogens. Therefore, the inventors screened the Prestwick library to identify antagonizing compounds to erythromycin or doxycycline on two abundant and prevalent gut microbes, *P. vulgatus* and *B. uniformis.*

*Preparation of screening plates.* The Prestwick Chemical Library was purchased from Prestwick Chemical Inc. and drugs were re-arrayed, diluted and stored in 96 well format as described before. The inventors prepared drug plates (2 x drug concentration) in MGAM medium and stored them at -30°C. For each experiment, drug plates were thawed, supplemented with the respective antibiotic solution (freshly prepared in MGAM) and pre-reduced in the anaerobic chamber overnight. All rearranging and aliquoting steps were done using the Biomek FXP (Beckman Coulter) system.

*Inoculation and screening conditions.* Strains were grown twice overnight, the second overnight culture was diluted in MGAM to reach OD_{578 nm} 0.04 (4 x the desired starting OD). 25 µl of the diluted cultures were used to inoculate wells containing 50 µl of 2x concentrated Prestwick drug and 25 µl of the 4x concentrated antibiotic using the semi-automated, 96-well multi-channel pipette epMotion96 (Eppendorf). Each well contained 1% DMSO, 20 µM of the Prestwick drug and a species-specific antibiotic concentration that was just inhibitory for the respective strain (0.625 µM for erythromycin, 0.04 µM doxycycline for *B. uniformis* and 0.08 µM doxycycline for *P. vulgatus*)*.* Plates were sealed with breathable membranes (Breathe-Easy^{®}) and OD₅₇₈ was measured hourly after 30 sec of linear shaking with a microplate spectrophotometer (EON, Biotek) and an automated microplate stacker (Biostack 4, Biotek) fitted inside a custom-made incubator (EMBL Mechanical Workshop). Growth curves were collected up to 24 h. For each antibiotic, each species was screened in biological duplicates. All experiments included control wells of unperturbed growth (32 wells per run) and control wells for growth in the presence of the antibiotic only (8 wells per plate).

*Analysis pipeline and hit calling:* All growth curves within a plate were truncated at the transition time from exponential to stationary phase and converted to normalized AUCs using in-run control wells (no drug) as described before. The inventors then calculated z-scores based on these normalized AUCs, removed replicates with 8-fold differences in z-scores to eliminate noise effects, computed mean z scores across the two replicates and selected combinations with mean z-scores > 3. This selection included 19 potential antibiotic antagonists and the inventors followed up on 14 of them (7 potential erythromycin and 7 potential doxycycline antagonists in either *P. vulgatus* or *B. uniformis* - see Fig. 4) in independent experiments.

*Validation of microbiome-protective antagonists:* In a first step, the inventors kept the erythromycin/doxycycline concentration constant (0.625 µM for erythromycin, 0.078 µM (*P. vulgatus*)/ 0.039 µM (*B. uniformis*) for doxycycline) and tested concentration gradients of the potential antagonists with ranges depending on the antagonist's solubility. Compounds were purchased from independent vendors and dissolved at 100x starting concentration in DMSO. Eight 2-fold serial dilutions were prepared in 96-well plates with each row containing a different antagonist, sufficient control DMSO wells and wells with just the respective antibiotic ('antibiotic-only' control). These master plates were diluted in MGAM medium (50 µl) to 2 x assay concentration and 25 µl freshly prepared antibiotic solution (4x test concentration) was added. Plates were pre-reduced overnight in an anaerobic chamber and inoculated with 25 µl of overnight cultures (prepared as described under "Growth conditions") to reach a starting OD₅₇₈ of 0.01 and 1% DMSO concentration. Growth was monitored hourly for 24 h after 30 sec of linear shaking. Experiments were performed in biological triplicates. For analysis, growth curves were converted into normalized AUCs (see above). The inventors accounted for residual growth in the presence of the antibiotic by subtracting the median normalized AUCs of the 'antibiotic-only' control per plate. The inventors computed medians across triplicates and considered a normalized AUC > 0.25 as concentration-dependent growth rescue by the antagonist.

*Checkerboard assays for anaerobic commensals:* Validated antagonists were further investigated in 8x8 checkerboard assays, where both antibiotics and antagonists were titrated against each other. Such assays were first performed for the commensals that were originally screened (i. e. *P. vulgatus* and *B. uniformis* - 4 replicates) and later expanded towards 6 further gut microbes (*B. caccae, B. fragilis* NT, *B. ovatus, B. thetaiotaomicron, P. copri, P. distasonis -* 2 replicates). For vertical gradients, 2-fold serial dilutions of the antagonists were prepared first in 100x in DMSO and diluted in MGAM as described above (section 'Validation of microbiome-protective antagonists'). Horizontal antibiotic dilution series were freshly prepared in MGAM at 4x final concentration in equidistant concentration steps. Both, vertical and horizontal dilution series were combined (50 µl of the antagonist gradients (2x) and 25 µl of the antibiotic gradients (4x)) in 96 well plates. Plates were pre-reduced under anaerobic conditions overnight, inoculated with 25 µl of diluted overnight culture (at 4x starting OD) and sealed with breathable membrane (Breathe-Easy^{®}). Bacterial growth was monitored once per hour for 24 hours after 30 sec linear shaking (Eon + Biostack 4, Biotek) under anaerobic conditions. Growth curves were converted into normalized AUCs as described using in-plate controls to define unperturbed growth.

*Checkerboard assays for pathogens under aerobic conditions:* For three pathogens (*S. aureus* Newman ATCC 13420, *S. aureus* subsp. aureus Rosenbach 1884 DSM20231 and *E. faecium* ATCC19434) 8x8 checkerboard assays were performed in transparent 384 well plates (Greiner BioOne GmbH), with each well containing 30 µl (50 µl for *S. pneumoniae*) in total. *S. aureus* strains were grown in tryptic soy broth, *E. faecium* in BHI medium. Antagonistic drug concentrations were 2-fold distant. Cell were inoculated at initial OD₅₉₅ₙₘ ~0.01 from an overnight culture. Plates were sealed with breathable membranes (Breathe-Easy), incubated at 37°C (Cytomat 2, Thermo Scientific) with continuous shaking and OD₅₉₅ₙₘ was measured every 30 minutes for 16 hours in a Filtermax F5 multimode plate reader (Molecular Devices). Growth was evaluated at the transition to stationary phase (9 hours for *S. aureus,* 12 hours for *E. faecium*). All experiments were done at least in 2 biological replicates and 2 technical replicates. For *S. pneumoniae* D39, the inventors only tested concentration gradients of the potential antagonists in constant concentration of the antibiotics in BHI medium.

**Table 1: Screen for microbiome-protective antagonists to erythromycin/tetracycline**

| **Drug** | **Antibiotic** | **Strain** | **Mean z-score** | **Validation** |
|---|---|---|---|---|
| Hexestrol | Erythromycin | B.uniformis | 9.630 | TRUE |
| Diethylstilbestrol | Erythromycin | B.uniformis | 9.258 | TRUE |
| Famprofazone | Erythromycin | B.uniformis | 7.832 | FALSE |
| Tolfenamic acid | Erythromycin | B.vulgatus | 7.381 | TRUE |
| Dicumarol | Erythromycin | B.vulgatus | 7.313 | TRUE |
| Lorglumide sodium salt | Erythromycin | B.uniformis | 6.106 | TRUE |
| Benzbromarone | Erythromycin | B.uniformis | 6.034 | FALSE |
| Iopanoic acid | Doxycycline | P.vulgatus | 5.698 | TRUE |
| Tiratricol, 3,3',5-triiodothyroacetic acid | Doxycycline | B.uniformis | 4.333 | TRUE |
| Tolfenamic acid | Doxycycline | P.vulgatus | 4.000 | TRUE |
| Losartan | Doxycycline | B.uniformis | 3.421 | FALSE |
| Diethylstilbestrol | Doxycycline | P.vulgatus | 3.296 | NA |
| Celecoxib | Doxycycline | B.uniformis | 3.275 | FALSE |
| Diflunisal | Doxycycline | P.vulgatus | 3.265 | TRUE |
| Efavirenz | Doxycycline | P.vulgatus | 3.138 | NA |
| Diclazuril | Doxycycline | P.vulgatus | 3.117 | NA |
| Triclosan | Doxycycline | B.uniformis | 3.066 | FALSE |
| Clonixin Lysinate | Doxycycline | P.vulgatus | 3.061 | NA |
| Meclofenamic acid sodium salt monohydrate | Doxycycline | P.vulgatus | 3.010 | NA |

| | | | | |
|---|---|---|---|---|
| Abbreviation: NA = not available | | | | |

### Example 3: Validation of microbiome-protective antibiotic antagonisms

Of the 19 identified hits (Fig. 3 b, Table 1), the inventors tested the 14 candidates with the strongest activity in a concentration-dependent manner (Fig. 5 a). Nine retained antagonistic effects over a broader concentration range, which the inventors confirmed by checkerboard assays (Fig. 3 c). The antidotes that showed the strongest antagonisms were the anticoagulant drug dicumarol, and two non-steroidal anti-inflammatory drugs, tolfenamic acid and diflusinal. While dicumarol rescued *P. vulgatus* from erythromycin and diflusinal from doxycycline, tolfenamic acid was able to protect *P. vulgatus* from both drugs.

In addition, these interactions were able to at least partially rescue the killing of *P. vulgatus* by erythromycin and doxycycline (Fig. 5 b). The inventors then probed these 3 drugs for their ability to protect other abundant gut commensals and confirmed that both dicumarol and tolfenamic acid were able to counteract erythromycin on several species (Fig. 3 d, Fig. 6). In contrast, both drugs did not affect the potency of erythromycin on *Staphylococcus aureus* and *Streptococcus pneumoniae,* pathogens against which erythromycin is routinely prescribed (Fig. 3 e, Fig. 7 a). For example, tolfenamic acid and dicumarol at concentration ranges of 5-40 µM could rescue the growth of 5/7 tested abundant gut commensal species at clinically relevant erythromycin concentrations (Fig. 3 f, Fig. 7 b). Altogether, the inventor's data provides antidotes that specifically mask the collateral damage of antibiotics on commensals.

**Example 4:** Representative species of the order Bacteroidales can be protected from the exemplary macrolide antibiotic azithromycin by dicumarol, benzbromarone or tolfenamic acid.

The inventors followed up on earlier findings, where it was shown that the marketed drugs dicumarol, benzbromarone and tolfenamic acid can rescue the growth of *P. vulgatus* in the presence of erythromycin. In particular, they aimed at testing, whether these antidotes can also protect from other macrolidessuch as azithromycin, a clinically more relevant antibiotic. It was generally found that all antidotes as tested worked at least equally well, in some cases even better, in antagonizing azithromycin compared to erythromycin (see Figure 8).

**Example 5:** Synthetic gut commensal communities can be protected from macrolide antibiotics by the antidotes dicumarol, benzbromarone or tolfenamic acid.

So far, antidote effects have only been analyzed in single or pure cultures. However, the human gut microbiome is a complex community of many different bacterial taxa that is usually not adequately mimicked in monocultures. Hence, the inventors created a synthetic gut commensal community consisting of P. vulgatus,B. fragilis nt, B. thetaiotaomicron, P. copri, B. caccae, B. ovatus, P. distasonis, and tested antidote effects in this setting. Growth of the community is rescued from both erythromycin and azithromycin with all three antidotes as tested, as was observed in pure culture, the rescue from azithromycin is stronger than from erythromycin (see Figure 9).

**Example 6:** Human intestinal pathogensof the genus enterococcus are not rescued from macrolide antibiotics by the antidotes dicumarol, benzbromarone or tolfenamic acid.

Here, the inventors expanded testing towards additional antidotes, and included the intestinal human pathogen Enterococcus faecalis. Neither dicumarol nor benzbromarone or tolfenamic acid could rescue E. faecalis/E. faecium from erythromycin or azithromycin treatment. Moreover, the antidote benzbromarone and tolfenamic acid synergized with the antibacterial effects of erythromycin/azithromycin against E. faecalis/E. faecium (see Figure 10).

**Example 7**: In Enterococci-containing synthetic communities, the antidotes dicumarol and benzbromarone selectively rescue growth of commensal bacteria from erythromycin treatment, while pathogenic enterococci are inhibited.

The inventors exposed syntheticcommunities that contained the intestinal gut pathogen E. faecalis to erythromycin, and evaluated rescue of gut commensals and simultaneous killing of the pathogen in the presence of antidotes. For both, benzbromarone and dicumarol, the inventors could show rescue of the community despite erythromycin treatment, while E. faecalis cells were efficiently inhibited. This result shows that within microbial communities, the antidotes can selectively rescue commensals from erythromycin (see Figure 11). As an example how to interpret the plots in Figure 11, if focused on an erythromycin concentration of 1.15 µM, the community as analyzed is partially inhibited, as the tiles of the heat map indicate in pale shading. The community still contains ~9 x 10⁵ E. faecalis cells. If then 40 µM dicumarol or 20 µM benzbromarone is added, the community is rescued, as the AUCs are higher/tiles are filled in darker shading color. However, E. faecalis counts drop/are still found at below the detection limit. Thus, only non-pathogenic community members are rescued in this assay.

## Claims

1. Dicumarol, or a pharmaceutically acceptable salt thereof, for use in treating dysbiosis and in antagonizing the antibacterial effect of at least one macrolide antibiotic, such as erythromycin, in *Phocaeicola,* such as, for example, *P. vulgatus,*
and wherein said dicumarol, or the pharmaceutically acceptable salt thereof, does not antagonize the antibacterial effect of said at least one macrolide antibiotic, such as erythromycin, in at least one second pathogenic bacterium, such as, for example, *S*. *aureus* or *S. pneumoniae.*

2. A pharmaceutical composition, comprising:
dicumarol, or a pharmaceutically acceptable salt thereof and at least one macrolide antibiotic, such as erythromycin,
for use in treating dysbiosis, and in antagonizing the antibacterial effect of the at least one macrolide antibiotic, such as erythromycin, in *Phocaeicola,* such as, for example, *P. vulgatus,*
and wherein said dicumarol, or the pharmaceutically acceptable salt thereof, does not antagonize the antibacterial effect of said at least one macrolide antibiotic, such as erythromycin, in at least one second pathogenic bacterium, such as, for example, *S*. *aureus* or *S. pneumoniae.*

3. The pharmaceutical composition for use according to claim 2, further comprising a pharmaceutically acceptable additive, carrier, diluent, solvent, filter, lubricant, excipient, binder, and/or stabilizer.

4. The pharmaceutical composition for use according to claim 2 or 3, wherein said macrolide antibiotic is selected from the group consisting of erythromycin, azithromycin, clarithromycin, dirithromycin, or clindamycin, and a pharmaceutically acceptable salt thereof.

5. The compound for use according to claim 1, or the pharmaceutical composition for use according to any one of claims 2 to 4, wherein said at least one second bacterium is a Gram-positive bacterium or a Gram-negative bacterium, and wherein said at least one second bacterium is a member of a genus selected from *Enterococcus, Staphylococcus, Enterobacter, Streptococcus, Pseudomonas, Escherichia, Salmonella, Helicobacter, Neisseria, Campylobacter, Chlamydia, Clostridia, Citrobacter, Vibrio, Treponema, Mycobacterium, Klebsiella, Actinomyces, Bacteroides, Bordetella, Borrelia, Brucella, Corynebacteria, Diplococcus, Fusobacterium, Leptospira, Listeria, Pasteurella, Proteus, Rickettsia, Shigella, Yersinia, Parabacteroides, Odoribacter, Faecalibacteria, Collinsella, Eggerthella, Lactonifactor, Pediococcus, Leuconostoc, Lactococcus, Roseburia, Coliform, Bacillus, Franscicella, Acinetobacter, Legionella, Actinobacillus, Coxiella, Kingella kingae, Haemophilus,* and combinations thereof, optionally wherein said at least one second bacterium is an antibiotic-resistant bacterium and/or a multi-drug resistant bacterium.

6. The compound for use according to claim 1, or the pharmaceutical composition for use according to any one of claims 2 to 5, wherein said at least one second compound is for use in the prevention and/or treatment of a bacterial infection, preferably wherein said bacterial infection is selected from an infection of the gastrointestinal tract, an infection of the urogenital tract, an infection of the upper and lower respiratory tract, rhinitis, tonsillitis, pharyngitis, bronchitis, pneumonia, an infection of the inner organs, nephritis, hepatitis, peritonitis, endocarditis, meningitis, osteomyelitis, an infection of the eyes, an infection of the ears, a cutaneous infection, a subcutaneous infection, an infection after burn, diarrhea, colitis, pseudomembranous colitis, a skin disorder, toxic shock syndrome, bacteremia, sepsis, pelvic inflammatory disease, an infection of the central nervous system, wound infection, intra-abdominal infection, intravascular infection, bone infection, joint infection, acute bacterial otitis media, pyelonephritis, deep-seated abscess, and tuberculosis.

7. The compound for use according to claim 1, or the pharmaceutical composition for use according to any one of claims 2 to 6, wherein said compound or said pharmaceutical composition is in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, or room spray, optionally wherein said components (i) and (ii) of said pharmaceutical composition are administered to a subject simultaneously, separately, or sequentially.

8. The compound for use according to claim 1, or the pharmaceutical composition for use according to any one of claims 2 to 7, wherein said subject is a mammal, such as a mouse, rat, guinea pig, rabbit, cat, dog, monkey, or preferably a human, such as a human patient, optionally wherein said compound or said pharmaceutical composition is administered to said subject in a therapeutically effective amount.

## Patentansprüche

1. Dicumarol oder ein pharmazeutisch verträgliches Salz davon zur Verwendung bei der Behandlung von Dysbiose und zur Aufhebung der antibakteriellen Wirkung mindestens eines Makrolid-Antibiotikums, wie beispielsweise Erythromycin, bei *Phocaeicola*, wie beispielsweise *P. vulgatus*,
wobei das Dicumarol oder das pharmazeutisch verträgliche Salz davon die antibakterielle Wirkung des mindestens einen Makrolid-Antibiotikums, wie beispielsweise Erythromycin, in mindestens einem zweiten pathogenen Bakterium, wie beispielsweise *S. aureus* oder *S. pneumoniae*, nicht antagonisiert.

2. Pharmazeutische Zusammensetzung, umfassend:
Dicumarol oder ein pharmazeutisch verträgliches Salz davon und mindestens ein Makrolid-Antibiotikum, wie beispielsweise Erythromycin,
zur Verwendung bei der Behandlung von Dysbiose und zur Hemmung der antibakteriellen Wirkung des mindestens einen Makrolid-Antibiotikums, wie beispielsweise Erythromycin, in *Phocaeicola*, wie beispielsweise *P. vulgatus*,
und wobei das Dicumarol oder das pharmazeutisch verträgliche Salz davon die antibakterielle Wirkung des mindestens einen Makrolid-Antibiotikums, wie Erythromycin, in mindestens einem zweiten pathogenen Bakterium, wie beispielsweise *S. aureus* oder *S. pneumoniae*, nicht antagonisiert.

3. Pharmazeutische Zusammensetzung zur Verwendung gemäß Anspruch 2, die ferner einen pharmazeutisch verträglichen Zusatzstoff, Träger, Verdünnungsmittel, Lösungsmittel, Filter, Gleitmittel, Hilfsstoff, Bindemittel und/oder Stabilisator umfasst.

4. Pharmazeutische Zusammensetzung zur Verwendung gemäß Anspruch 2 oder 3, wobei das Makrolid-Antibiotikum aus der Gruppe ausgewählt ist, die aus Erythromycin, Azithromycin, Clarithromycin, Dirithromycin oder Clindamycin und einem pharmazeutisch verträglichen Salz davon besteht.

5. Die Verbindung zur Verwendung gemäß Anspruch 1 oder die pharmazeutische Zusammensetzung zur Verwendung gemäß einem der Ansprüche 2 bis 4, wobei das mindestens eine zweite Bakterium ein grampositives Bakterium oder ein gramnegatives Bakterium ist und wobei das mindestens eine zweite Bakterium ein Mitglied einer Gattung ist, ausgewählt aus *Enterococcus*, *Staphylococcus, Enterobacter, Streptococcus, Pseudomonas, Escherichia, Salmonella, Helicobacter, Neisseria, Campylobacter, Chlamydia, Clostridia, Citrobacter, Vibrio, Treponema, Mycobacterium, Klebsiella, Actinomyces, Bacteroides, Bordetella, Borrelia, Brucella, Corynebacteria, Diplococcus, Fusobacterium, Leptospira, Listeria, Pasteurella, Proteus, Rickettsia, Shigella, Yersinia, Parabacteroides, Odoribacter, Faecalibacteria, Collinsella, Eggerthella, Lactonifactor, Pediococcus, Leuconostoc, Lactococcus, Roseburia, Coliforme, Bacillus, Francisella, Acinetobacter, Legionella, Actinobacillus, Coxiella, Kingella kingae, Haemophilus* und Kombinationen davon, wobei das mindestens eine zweite Bakterium gegebenenfalls ein antibiotikaresistentes Bakterium und/oder ein multiresistentes Bakterium ist.

6. Die Verbindung zur Verwendung gemäß Anspruch 1 oder die pharmazeutische Zusammensetzung zur Verwendung gemäß einem der Ansprüche 2 bis 5, wobei die mindestens eine zweite Verbindung zur Verwendung bei der Vorbeugung und/oder Behandlung einer bakteriellen Infektion bestimmt ist, vorzugsweise wobei die bakterielle Infektion ausgewählt ist aus einer Infektion des Magen-Darm-Trakts, einer Infektion des Urogenitaltrakts, einer Infektion der oberen und unteren Atemwege, Rhinitis, Tonsillitis, Pharyngitis, Bronchitis, Pneumonie, einer Infektion der inneren Organe, Nephritis, Hepatitis, Peritonitis, Endokarditis, Meningitis, Osteomyelitis, einer Infektion der Augen, einer Infektion der Ohren, einer Hautinfektion, einer subkutanen Infektion, einer Infektion nach Verbrennungen, Durchfall, Kolitis, pseudomembranöse Kolitis, eine Hauterkrankung, das toxische Schocksyndrom, Bakteriämie, Sepsis, eine entzündliche Erkrankung des Beckens, eine Infektion des Zentralnervensystems, eine Wundinfektion, eine intraabdominale Infektion, eine intravaskuläre Infektion, eine Knocheninfektion, eine Gelenkinfektion, eine akute bakterielle Mittelohrentzündung, Pyelonephritis, ein tiefsitzender Abszess und Tuberkulose.

7. Die Verbindung zur Verwendung gemäß Anspruch 1 oder die pharmazeutische Zusammensetzung zur Verwendung gemäß einem der Ansprüche 2 bis 6, wobei die Verbindung oder die pharmazeutische Zusammensetzung in flüssiger, trockener oder halbfester Form vorliegt, wie beispielsweise in Form einer Tablette, einer Filmtablette, einer Brausetablette, einer Kapsel, eines Pulvers, eines Granulats, einer Zuckertablette, eines Lutschtabletten, Pille, Ampulle, Tropfen, Zäpfchen, Emulsion, Salbe, Gel, Tinktur, Paste, Creme, feuchte Kompresse, Gurgellösung, Pflanzensaft, Nasenspray, Inhalationsgemisch, Aerosol, Mundwasser, Mundspray, Nasenspray oder Raumspray vorliegt, wobei die Komponenten (i) und (ii) der pharmazeutischen Zusammensetzung einem Patienten wahlweise gleichzeitig, getrennt oder nacheinander verabreicht werden.

8. Die Verbindung zur Verwendung gemäß Anspruch 1 oder die pharmazeutische Zusammensetzung zur Verwendung gemäß einem der Ansprüche 2 bis 7, wobei das Subjekt ein Säugetier ist, wie beispielsweise eine Maus, Ratte, Meerschweinchen, Kaninchen, Katze, Hund, Affe oder vorzugsweise ein Mensch, wie beispielsweise ein menschlicher Patient, wobei die Verbindung oder die pharmazeutische Zusammensetzung dem Subjekt gegebenenfalls in einer therapeutisch wirksamen Menge verabreicht wird.

## Revendications

1. Dicumarol, ou sel pharmaceutiquement acceptable de celui-ci, utilisable pour traiter une dysbiose et pour antagoniser l'effet antibactérien d'au moins un antibiotique macrolide, tel que l'érythromycine, chez un *Phocaeicola,* tel que, par exemple, *P. vulgatus,* et dans lequel ledit dicumarol, ou le sel pharmaceutiquement acceptable de celui-ci, n'antagonise pas l'effet antibactérien dudit au moins un antibiotique macrolide, tel que l'érythromycine, chez au moins une seconde bactérie pathogène, telle que, par exemple, *S. aureus* ou *S. pneumoniae.*

2. Composition pharmaceutique comprenant :
du dicumarol, ou un sel pharmaceutiquement acceptable de celui-ci, et au moins un antibiotique macrolide, tel que l'érythromycine, utilisable pour traiter une dysbiose et pour antagoniser l'effet antibactérien d'au moins un antibiotique macrolide, tel que l'érythromycine, chez un *Phocaeicola,* tel que, par exemple, *P. vulgatus,* et dans laquelle ledit dicumarol, ou le sel pharmaceutiquement acceptable de celui-ci, n'antagonise pas l'effet antibactérien dudit au moins un antibiotique macrolide, tel que l'érythromycine, chez au moins une seconde bactérie pathogène, telle que, par exemple, *S. aureus* ou *S. pneumoniae.*

3. Composition pharmaceutique pour utilisation selon la revendication 2, comprenant en outre un additif, support, diluant, solvant, filtre, lubrifiant, excipient, liant et/ou stabilisant, pharmaceutiquement acceptable.

4. Composition pharmaceutique pour utilisation selon la revendication 2 ou 3, dans laquelle ledit antibiotique macrolide est choisi dans le groupe constitué d'érythromycine, azithromycine, clarithromycine, dirithromycine ou clindamycine, et un sel pharmaceutiquement acceptable de celles-ci

5. Composé pour utilisation selon la revendication 1, ou composition pharmaceutique pour utilisation selon l'une quelconque des revendications 2 à 4, dans lequel ladite au moins une seconde bactérie est une bactérie à gram-positif ou une bactérie à gram-négatif, et dans lequel ladite au moins une seconde bactérie est un membre d'un genre choisi parmi *Enterococcus, Staphylococcus, Enterobacter, Streptococcus, Pseudomonas, Escherichia, Salmonella, Helicobacter, Neisseria, Campylobacter, Chlamydia, Clostridia, Citrobacter, Vibrio, Treponema, Mycobacterium, Klebsiella, Actinomyces, Bacteroides, Bordetella, Borrelia, Brucella, Corynebacteria, Diplococcus, Fusobacterium, Leptospira, Listeria, Pasteurella, Proteus, Rickettsia, Shigella, Yersinia, Parabacteroides, Odoribacter, Faecalibacteria, Collinsella, Eggerthella, Lactonifactor, Pediococcus, Leuconostoc, Lactococcus, Roseburia, Coliform, Bacillus, Franscicella, Acinetobacter, Legionella, Actinobacillus, Coxiella, Kingella kingae, Haemophilus,* et des combinaisons de ceux-ci, facultativement dans lequel ladite au moins une seconde bactérie est une bactérie résistante aux antibiotiques et/ou une bactérie multirésistante.

6. Composé pour utilisation selon la revendication 1, ou composition pharmaceutique pour utilisation selon l'une quelconque des revendications 2 à 5, dans lequel ledit au moins un second composé est destiné à être utilisé dans la prévention et/ou le traitement d'une infection bactérienne, de préférence dans lequel ladite infection bactérienne est choisie parmi infection de l'appareil gastrointestinal, infection de l'appareil urogénital, infection des voies respiratoires supérieures et inférieures, rhinite, amygdalite, pharyngite, bronchite, pneumonie, infection des organes internes, néphrite, hépatite, péritonite, endocardite, méningite, ostéomyélite, infection des yeux, infection des oreilles, infection cutanée, infection sous-cutanée, infection consécutive à une brûlure, diarrhée, colite, colite pseudo-membraneuse, trouble cutané, syndrome de choc toxique, bactériémie, septicémie, maladie inflammatoire pelvienne, infection du système nerveux central, infection de plaie, infection intra-abdominale, infection intravasculaire, infection osseuse, infection articulaire, otite moyenne bactérienne aiguë, pyélonéphrite, abcès profond et tuberculose.

7. Composé pour utilisation selon la revendication 1, ou composition pharmaceutique pour utilisation selon l'une quelconque des revendications 2 à 6, dans lequel ledit composé ou ladite composition pharmaceutique est sous forme liquide, sèche ou semi-solide, telle que, par exemple, sous la forme d'un comprimé, d'un comprimé enrobé, d'un comprimé effervescent, d'une gélule, d'une poudre, d'un granulé, d'un comprimé enrobé de sucre, d'une pastille, d'une pilule, d'une ampoule, d'une goutte, d'un suppositoire, d'une émulsion, d'une pommade, d'un gel, d'une teinture, d'une pâte, d'une crème, d'une compresse humide, d'une solution pour gargarisme, d'un jus de plante, d'un agent nasal, d'un mélange pour inhalation, d'un aérosol, d'un bain de bouche, d'un spray buccal, d'un spray nasal ou d'un spray d'ambiance, facultativement dans lequel lesdits composants (i) et (ii) de ladite composition pharmaceutique sont administrés à un sujet simultanément, séparément ou séquentiellement.

8. Composé pour utilisation selon la revendication 1, ou composition pharmaceutique pour utilisation selon l'une quelconque des revendications 2 à 7, dans lequel ledit sujet est un mammifère, tel qu'une souris, un rat, un cobaye, un lapin, un chat, un chien, un singe, ou de préférence un humain, tel qu'un patient humain, facultativement dans lequel ledit composé ou ladite composition pharmaceutique est administré audit sujet en une quantité thérapeutiquement efficace.
